# EUROPEAN PATENT APPLICATION

(11) **EP 3 756 624 A2**
(43) Date of publication of application: **30.12.2020**
(21) Application number: 20180385.5
(22) Date of filing: 16.06.2020
(51) Int. Cl.: A61F 2/30, C09D 5/14, C09D 7/61, C08K 3/08, C08K 3/32

(54) **ION INCORPORATED PLASMA SPRAYED HYDROXYAPATITE COATINGS AND METHOD OF MAKING THE SAME**

(30) Priority: 28.06.2019 US 201916457173
(71) Applicant: DePuy Synthes Products, Inc., Raynham, Massachusetts 02767-0350 (US)
(72) Inventor: KASINATH, RAJENDRA, WARSAW, INDIANA 46582 (US); QU, HAIBO, WARSAW, INDIANA 46582 (US)
(74) Representative: Pawlyn, Anthony Neil

(57) **Abstract**

A coated implant includes a coating comprising a doped hydroxyapatite portion and an undoped hydroxyapatite portion. The coated implant can be formed by treating a hydroxyapatite coating with a solution comprising a dopant metal.

## Description

### TECHNICAL FIELD

The present disclosure relates generally to a coated surface, and more particularly to a hydroxyapatite coated surface.

### BACKGROUND

Bone repair often involves the use of orthopaedic implants to replace missing bone or to support bone during the healing process. It is typically desirable to coat such orthopaedic implants with osteoconductive materials to encourage bone growth or biological fixation.

Hydroxyapatite (HA) is a naturally occurring mineral found in bones and teeth. Studies have shown that HA is osteoconductive, and orthopaedic implants have been coated with HA for this reason.

Various processes for coating implants with HA are known. One process used for coating implants is plasma spray. In this process, HA powder is fed into a high temperature torch with a carrier gas. The HA powder is partially melted and then impacts the substrate at high velocity whereupon it is rapidly quenched back to room temperature.

Incorporating dopant metals into hydroxyapatite coatings has also been reported. To incorporate the dopant metal into the hydroxyapatite coating, the dopant metal is mixed with hydroxyapatite prior to forming the coating. Although typically only the surface of the formed coating is active, such a method uniformly distributes the dopant metal throughout the hydroxyapatite coating. Such a method also requires a user prepare a specific dopant metal/hydroxyapatite composition for each formed coating.

### SUMMARY

A coated orthopaedic implant comprises a substrate having a bone-facing surface and a coating located on the bone-facing surface of the substrate. In illustrative embodiments, the coating comprises hydroxyapatite.

In some embodiments, the coating is a doped coating. In illustrative embodiments, the doped coating comprises calcium and a dopant metal. In some embodiments, the concentration of the dopant metal is anisotropic in the doped coating. In some embodiments, the doped coating comprises at least two dopant metals. In some embodiments, the dopant metal is selected from the group consisting of magnesium, strontium, gallium, zinc, copper, silver, europium, terbium, and combinations thereof.

In some embodiments, the doped coating comprises a doped portion and an undoped portion located between the doped portion and the substrate. In some embodiments, the doped portion comprises a dopant metal. In some embodiments, the undoped portion is free or substantially free of the dopant metal.

According to another aspect, a process for forming a coated implant comprises a step of contacting a coating and a step of washing. In some embodiments, the step of contacting is performed by contacting a hydroxyapatite coating on a substrate with an aqueous solution comprising a dopant metal ion. In some embodiments, the step of washing the hydroxyapatite coating forms the doped hydroxyapatite coating comprising calcium and a dopant metal.

In some embodiments, the concentration of the dopant metal is anisotropic in the doped hydroxyapatite coating. In some embodiments, the doped hydroxyapatite coating has improved antibacterial properties compared to the hydroxyapatite coating. In some embodiments, the improvement is at least 10-fold. In some embodiments, the improvement is at least 1,000-fold.

In some embodiments, the percent crystallinity of the doped hydroxyapatite coating is higher than the percent crystallinity of the hydroxyapatite coating. In some embodiments, the percent crystallinity of the doped hydroxyapatite coating is at least about 93% or at least about 95%.

According to another aspect, a coated implant comprises a substrate and a coating on a surface of the substrate.

In some embodiments, the coating includes a doped hydroxyapatite portion and an undoped hydroxyapatite portion. Illustratively, the undoped hydroxyapatite portion is located between the doped hydroxyapatite portion and the substrate. In some embodiments, the doped hydroxyapatite portion comprises calcium and a dopant metal. In some embodiments, the undoped hydroxyapatite portion does not include the dopant metal.

### BRIEF DESCRIPTION OF THE DRAWINGS

FIG. 1 is a perspective view of an orthopaedic prosthesis.
FIG. 2 is a diagrammatic view of a bone-facing surface of the orthopaedic prosthesis of FIG. 1 coated with a doped hydroxyapatite coating.
FIG. 3 shows a substrate coupon coated with Plasma-Sprayed Hydroxyapatite (PSHA).
FIG. 4 shows an SEM image of a PSHA coated substrate after treatment with a phosphate-zinc (PiZn) solution.
FIG. 5 shows the EDX pattern of the PiZn-treated PSHA coating.
FIG. 6 shows an XRD pattern of an untreated PSHA coating (denoted PSHA), a PSHA coating after treatment with a phosphate-gallium (PiGa) solution (denoted Ga HT) and a PiZn-treated PSHA coating (denoted Zn HT).
FIG. 7 shows an SEM image of a PiGa-treated PSHA coating.
FIG. 8 shows an EDX pattern of a PiGa-treated PSHA coating.
FIG. 9 shows an SEM image of the PSHA coating after treatment with a copper (Cu) solution.
FIG. 10 shows an EDX pattern of a PSHA coating after treatment with a Cu solution.
FIG. 11 shows an EDX pattern of a PSHA coating after treatment with a Cu solution.
FIG. 12 shows an XRD pattern of an untreated PSHA coating (denoted PSHA) and a Cu-treated PSHA coating (denoted Cu).
FIG. 13 shows an EDX pattern of a PSHA coating after treatment with a Eu solution.
FIG. 14 shows an EDX pattern of a PSHA coating after treatment with a Eu solution.
FIG. 15 shows an XRD pattern of an untreated PSHA coating (denoted PSHA) and a PSHA coating treated with a Eu solution (denoted Eu).
FIG. 16 shows an EDX pattern of a PSHA coating treated with a silver (Ag) solution.
FIG. 17 shows an EDX pattern of a PSHA coating treated with a Ag solution.
FIG. 18 shows an EDX pattern of a PSHA coating treated with a Ag solution.
FIG. 19 shows an XRD pattern of an untreated PSHA coating (denoted PSHA) and PSHA coatings treated with a Ag solution under different conditions (denoted Ag-2, Ag-3, and Ag-4).
FIG. 20 shows an EDX pattern of a PSHA coating after treatment with a PiZn solution and a Cu solution.
FIG. 21 shows an EDX pattern of a PSHA coating after treatment with a Cu solution and a PiZn solution.
FIG. 22 shows an SEM image of a PSHA coating after a Zn solution-Cu solution treatment.
FIG. 23 shows an SEM image of a PSHA coating after treatment with a Cu solution and a Zn solution.
FIG. 24 shows an XRD pattern of an untreated PSHA coating (denoted PSHA) and a PSHA coating after treatment with a Zn solution and a Cu solution (denoted Zn-Cu).

### DETAILED DESCRIPTION OF THE DRAWINGS

While the concepts of the present disclosure are susceptible to various modifications and alternative forms, specific exemplary embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

References in the specification to "one embodiment," "an embodiment," "an example embodiment," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described.

Terms representing anatomical references, such as anterior, posterior, medial, lateral, superior, inferior, etcetera, may be used throughout the specification in reference to the orthopaedic implants or prostheses described herein as well as in reference to the patient's natural anatomy. Such terms have well-understood meanings in both the study of anatomy and the field of orthopaedics. Use of such anatomical reference terms in the written description and claims are intended to be consistent with their well-understood meanings unless noted otherwise.

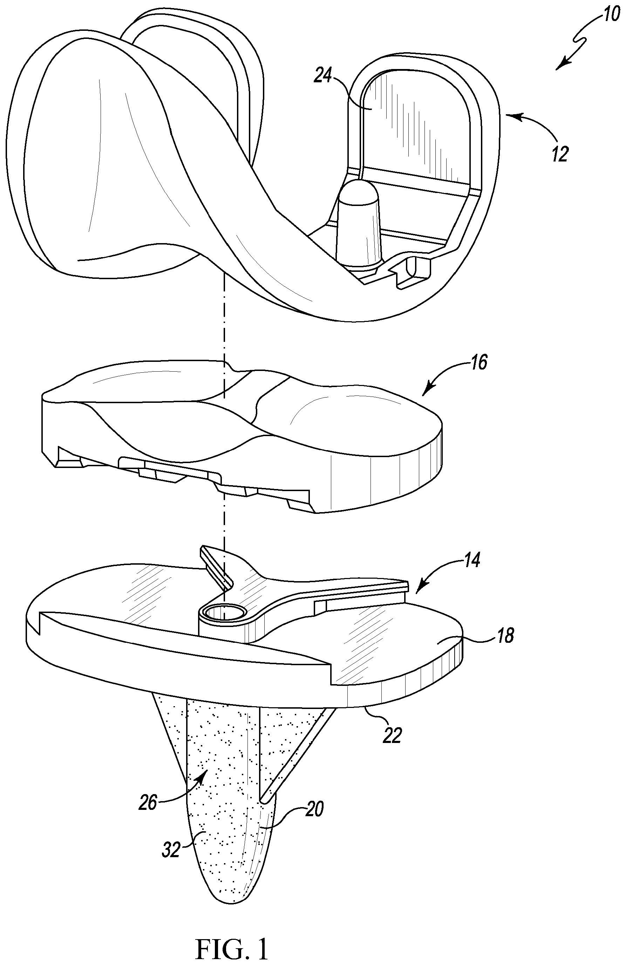

Referring now to Fig. 1, an exemplary knee prosthesis 10 is shown. The knee prosthesis 10 includes a femoral component 12, a tibial tray component 14, and an insert component 16. The tibial tray 14 includes a plate or platform 18 and an elongated stem 20 that extends away from the distal, bone-facing surface 22 of the platform 18. The elongated tibial stem 20 is configured to be implanted into a surgically-prepared proximal surface a patient's tibia. It should be appreciated that other fixation members, such as one or more short pegs or posts, may be used in lieu of the elongated stem 20. In the illustrative embodiment, the elongated tibial stem 20 includes an outer surface having a surface roughness (Ra) in a range of 3 microns to 7 microns.

The insert component 16 is securable to the tibial tray 14 via a snap-fit in the illustrative embodiment. In such a way, the insert 16 is fixed relative to the tibial tray 14 (i.e., it is not rotatable or moveable in the anterior/posterior or medial/lateral directions). It should be appreciated that in other embodiments the insert may be moveable relative to the tibial tray. It should also be appreciated that in other embodiments the tray and insert may be combined in a single, monolithic tibial component/

The femoral component 12 is configured to be implanted into a surgically prepared end of the patient's femur, and is configured to emulate the configuration of the patient's natural femoral condyles. The femoral component 12 comprises a bone surface 24, which is configured to contact the femur bone when implanted.

The components of the knee prosthesis 10 that engage the natural bone, such as the femoral component 12 and the tibial tray 14, may be constructed with a biocompatible metal, such as a cobalt chrome alloy, although other materials may also be used. The bone facing or engaging surfaces of these components, such as the distal, bone-facing surface 22 and the tibial stem 20 and the bone-facing surface 24 of the femoral component 12, may be textured to facilitate cementing the component to the bone, as described in greater detail below. Such bone facing or engaging surfaces may be coated with a hydroxyapatite coating or a doped hydroxyapatite coating 26, as shown in Figs. 1 2.

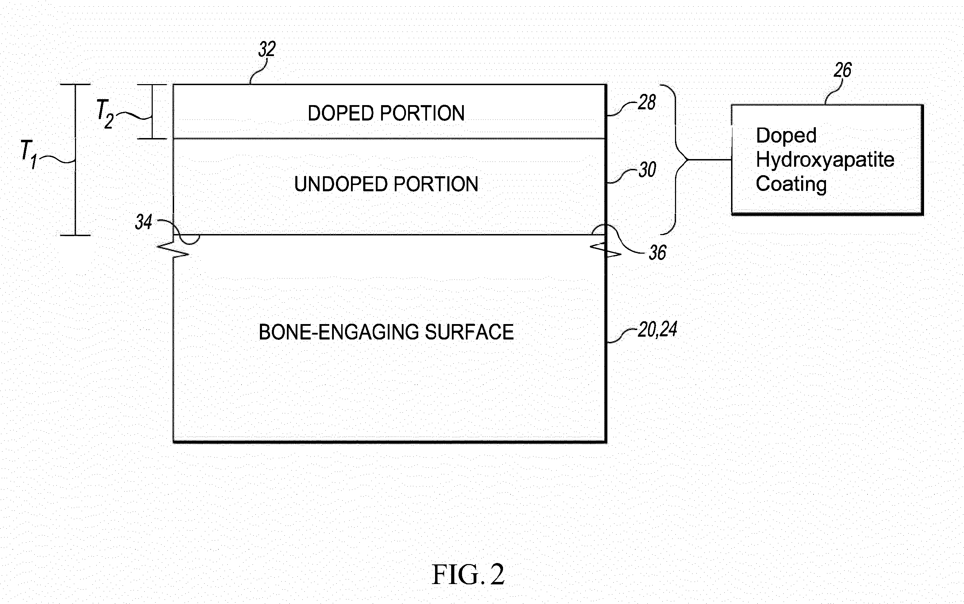

The present disclosure relates to implants such as orthopaedic prosthesis 10, and methods of making the same. Portions of the implants, such as the bone facing surfaces, may be coated with hydroxyapatite (HA), as shown in Fig. 2, which includes a doped hydroxyapatite coating 26. Throughout this disclosure, a coated substrate comprising a substrate (such as, for example, the bone-facing or engaging surfaces of the prosthesis 10) and a doped hydroxyapatite coating 26 are described in greater detail. In illustrative embodiments, the doped hydroxyapatite coating 26 comprises a doped portion 28 and an undoped portion 30, as shown in Fig. 2. The doped portion 28 is arranged to form an outer surface 32 of the doped hydroxyapatite coating 26.

An illustrative process of forming the orthopaedic prosthesis 10 includes a step of forging a material for an implant to form a forged part. Portions of the forged part are then masked. In illustrative embodiments, the bone-engaging surfaces are not masked. The unmasked surfaces are then grit blasted to a surface roughness (Ra) in a range of 3 microns to 7 microns. In some embodiments, the grit-blasted surfaces are coated with plasma-sprayed hydroxyapatite (PSHA). In some embodiments, the PSHA coated part is then washed. As described herein, the PSHA coated surfaces can optionally undergo a process to incorporate a dopant metal into the PSHA coated surface. In some embodiments, the illustrative process produces an orthopaedic prosthesis 10 having a doped hydroxyapatite coating 26 on the surface of the orthopaedic prosthesis 10, as shown in Fig. 1. In some embodiments, the illustrative process produces an orthopaedic prosthesis 10 having a doped hydroxyapatite coating on the bone-engaging surfaces, such as the elongated stem 20 and the femoral surface 24, as shown in Figs. 1 and 2.

Although a knee prosthesis is shown in Fig. 1, the doped hydroxyapatite coatings described herein may be equally suitable for components of a hip prosthesis, a shoulder prosthesis, a bone plate, other prosthetic joint components, and other prosthetic implants for use in the body.

In illustrative embodiments, the doped hydroxyapatite coating comprises calcium and at least one dopant metal. The dopant metal may provide advantageous properties to the coating such as improving osseointegration, providing infection prophylaxis, providing antibacterial properties, or providing fluorescence. The dopant metal may be introduced into the hydroxyapatite coating through an ion-exchange process where lattice positions in the hydroxyapatite coating are replaced with the dopant metal. In illustrative embodiments, incorporating the dopant metal does not alter the crystalline structure of the hydroxyapatite coating, as determined by XRD.

The coating, such as doped hydroxyapatite coating 26, comprises an outer surface 32 and an inner surface 34 spaced apart from the outer surface 32, as shown in Fig. 2. The outer surface 32 of the coating is arranged to form an outer surface of the implant. The inner surface 34 that is arranged to contact the outer surface 36 of the substrate, such as the bone-facing surface, as shown in Fig. 2.

The coating on the substrate, such as doped hydroxyapatite coating 26, has a particular thickness, T1, as measured by the distance from the outer surface 32 of the coating to the inner surface 34 of the coating. Illustratively, the coating may be less than about 500, less than about 300 or less than about 250 microns thick. In some embodiments, the coating is about 50 to about 500, about 50 to about 300, about 50 to about 200, about 50 to about 150, or about 80 to about 150 microns thick.

The doped hydroxyapatite coating 26 comprises a doped portion 28 and an undoped portion 30, as shown in Fig. 2. The undoped portion 30 is arranged to extend between and interconnect the doped portion 28 and the bone-facing surface. The doped portion is arranged to form the outer surface 32 of the coating. In illustrative embodiments, the concentration of the dopant metal is anisotropic in the coating. In some embodiments, the doped portion 28 comprises the dopant metal. In some embodiments, the undoped portion 30 is free of or substantially free of the dopant metal.

As described herein, an undoped hydroxyapatite coating that has already been formed on a substrate can be contacted with a dopant metal ion to form a doped hydroxyapatite coating. In some embodiments, the dopant metal ion is in an aqueous solution. Illustratively, the dopant metal ion replaces lattice positions present in the undoped hydroxyapatite coating to form the doped hydroxyapatite coating. Such an exchange provides a higher concentration of the dopant metal at the outer surface of the coating relative to the inner surface of the coating.

In some embodiments, the concentration of the dopant metal is higher at the outer surface of the coating compared to the inner surface of the coating such that the concentration of the dopant metal ion is anisotropic in the coating. In some embodiments, the dopant metal is present only in the doped portion of the doped coating and is not present in the undoped portion of the coating. Illustratively, the concentration of the dopant metal decreases from the outer surface of the coating toward the surface of the substrate. In some embodiments, the doped portion has a thickness T2 that is about 1, about 5, or about 10 microns thick. In some embodiments, the doped portion is about 0.1 microns, about 0.5 microns, about 1 micron, about 2 microns, about 3 microns, about 4 microns, about 5 microns, about 6 microns, about 7 microns, about 8 microns, about 9 microns, or about 10 microns thick. In some embodiments, the doped portion is about 0.1 microns to about 10 microns, about 0.1 microns to about 6 microns, about 0.5 microns to about 6 microns, or about 0.5 microns to about 4 microns thick. Illustratively, the remainder of the doped coating is the undoped portion.

In some embodiments, the substrate is selected from a natural or a non-natural material. In some embodiments, the substrate comprises a combination of a natural and a non-natural material. In some embodiments, the substrate comprises a material selected from the group consisting of a metal, a plastic, bone, a rubber, a gel, a cellulosic material, and combinations thereof. In some embodiments, the metal is selected from the group consisting of titanium, cobalt, chromium, nickel, gold, silver, an alloy thereof, or a combination thereof. In some embodiments, the substrate may comprise, consist of, or consist essentially of a cobalt chromium alloy. In some embodiments, the substrate may comprise, consist of, or consist essentially of titanium.

In illustrative embodiments, the amount of calcium in the outer surface of the doped hydroxyapatite coating is less than the amount of calcium in the outer surface of the undoped hydroxyapatite coating prior to contacting the undoped hydroxyapatite coating with a dopant solution. Illustratively, an untreated PSHA coating may comprise about 33.5% to about 37.5% calcium and preferably about 35% calcium. In some embodiments, the outer surface of the doped hydroxyapatite coating comprises less than about 38%, less than about 35%, or less than about 33% calcium by weight of the coating. In some embodiments, the outer surface of the doped hydroxyapatite coating comprises about 33% to about 38% calcium by weight in the outer surface of the coating.

In some embodiments, a doped hydroxyapatite coating on the surface of the substrate comprises calcium and at least one dopant metal. In some embodiments, the dopant metal is selected from the group consisting of magnesium, strontium, gallium, zinc, copper, silver, europium, terbium, an alloy thereof, and combinations thereof. In some embodiments, the doped hydroxyapatite coating comprises at least two dopant metals. In some embodiments, the doped hydroxyapatite coating comprises a combination of two metals such as zinc and copper, zinc and magnesium, zinc and strontium, zinc and gallium, zinc and silver, zinc and europium, zinc and terbium, magnesium and strontium, magnesium and gallium, magnesium and copper, magnesium and silver, magnesium and europium, magnesium and terbium, strontium and gallium, strontium and copper, strontium and silver, strontium and europium, strontium and terbium, gallium and copper, gallium and silver, gallium and europium, gallium and terbium, copper and silver, copper and europium, copper and terbium, silver and europium, silver and terbium, or europium and terbium. In some embodiments, the coating comprises at least three dopant metals.

In some embodiments, the dopant metal is distributed along the outer surface of the doped hydroxyapatite coating. In some embodiments, the dopant metal is distributed on part of the outer surface of the doped hydroxyapatite coating. In some embodiments, one dopant metal is distributed along one part of the outer surface of the doped hydroxyapatite coating, and a second dopant metal is distributed along a second part of the outer surface of the hydroxyapatite coating. In some embodiments, the two dopant metals are located together (i.e., distributed together) within the same whole or part of the outer surface of the doped hydroxyapatite coating.

In illustrative embodiments, the doped portion of the doped hydroxyapatite coating comprises at least one dopant metal that is present in the doped portion of the doped hydroxyapatite in an amount of at least about 0.1% by weight of the doped portion or at least 0.5% by weight of the doped portion. In some embodiments, the doped portion comprises about 0.1%, about 0.5%, about 1%, about 1.5%, about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5%, about 10%, about 10.5%, about 11%, about 11.5%, about 12%, about 12.5%, about 13%, about 13.5%, about 14%, about 14.5%, about 15%, about 15.5%, about 16%, about 16.5%, about 17%, about 17.5%, about 18%, about 18.5%, about 19%, about 19.5%, about 20%, about 20.5%, about 21%, about 21.5%, about 22%, about 22.5%, about 23%, about 23.5%, about 24%, about 24.5%, about 25%, about 25.5%, about 26%, about 27%, about 28%, about 29%, or about 30% by weight at least one dopant metal. In a first set of ranges, the doped portion comprises at least one dopant metal in an amount ranging from about 0.1% to about 30%, about 0.1% to about 25%, about 0.25% to about 25%, about 0.5% to about 25%, about 1% to about 25%, about 1.5% to about 25%, about 10% to about 25%, about 12% to about 25%, about 15% to about 25%, about 18% to about 25%, or about 20% to about 25% by weight of the doped portion. In a second set of ranges, the doped portion comprises at least one dopant metal in an amount ranging from about 0.1% to about 20%, about 0.1% to about 15%, about 0.5% to about 15%, about 1% to about 15%, about 1% to about 10%, about 2% to about 10%, or about 2% to about 10% by weight of the doped portion. In a third set of ranges, the doped portion comprises at least one dopant metal present in an amount ranging from about 2% to about 5%, about 3% to about 6%, about 7% to about 18%, about 7% to about 11%, about 0.25% to about 1%, about 0.1% to about 3%, about 1% to about 4%, about 2% to about 3%, about 9% to about 10%, about 12% to about 13%, about 19% by weight to about 20%, or about 22% to about 23% by weight of the doped portion.

In some embodiments, the dopant metal is present in the doped portion in an amount of about 0.5% by weight of the doped portion. In some embodiments, the dopant metal is present in the doped portion of the doped hydroxyapatite coating in an amount of about 1.9% by weight of the doped portion. In some embodiments, the dopant metal is present in the doped portion of the doped hydroxyapatite coating in an amount of about 2.6% by weight of the doped portion. In some embodiments, the dopant metal is present in the doped portion of the doped hydroxyapatite coating in an amount of about 2.7% by weight of the doped portion. In some embodiments, the dopant metal is present in the doped portion of the doped hydroxyapatite coating in an amount of about 3.7% by weight of the doped portion. In some embodiments, the dopant metal is present in the doped portion of the doped hydroxyapatite coating in an amount of about 4.7% by weight of the doped portion. In some embodiments, the dopant metal is present in the doped portion of the doped hydroxyapatite coating in an amount of about 7.8% by weight of the doped portion. In some embodiments, the dopant metal is present in the doped portion of the doped hydroxyapatite coating in an amount of about 9.8% by weight of the doped portion. In some embodiments, the dopant metal is present in the doped portion of the doped hydroxyapatite coating in an amount of about 12.8% by weight of the doped portion. In some embodiments, the dopant metal is present in the doped portion of the doped hydroxyapatite coating in an amount of about 19.8% by weight of the doped portion. In some embodiments, the dopant metal is present in the doped portion of the doped hydroxyapatite coating in an amount of about 22.7% by weight of the doped portion.

In illustrative embodiments, the doped portion of the doped hydroxyapatite coating comprises a particular atomic percentage of at least one dopant metal. In some embodiments, the atomic percentage of the dopant metal in the doped portion of the doped hydroxyapatite coating is at least about 0.05% or at least about 1%. In some embodiments, the atomic percentage of the at least one dopant metal ion in the doped portion of the doped hydroxyapatite coating is about 0.05%, about 0.1%, about 0.15%, about 0.2%, about 0.25%, about 0.3%, about 0.35%, about 0.4%, about 0.45%, about 0.5%, about 0.55%, about 0.6%, about 0.65%, about 0.7%, about 0.75%, about 0.8%, about 0.85%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2% about 4.3% about 4.4% about 4.5%, about 4.6%, about 4.7%, about 4.8%, about 4.9%, about 5%, about 5.1%, about 5.2%, about 5.3%, about 5.4%, or about 5.5%. In a first set of ranges, the atomic percentage of the at least one dopant metal in the doped portion of the doped hydroxyapatite coating is in a range of about 0.05% to about 5.5%, about 0.05% to about 5.2%, about 0.5% to about 5.2%, about 1% to about 4%, about 0.5% to about 2%, about 0.1% to about 1%, about 0.1% to about 0.5%, about 0.5% to about 1%, about 1% to about 2%, about 2% to about 4%, or about 3.5% to about 4%. In a second set of ranges, the atomic percentage of the at least one dopant metal in the doped portion of the doped hydroxyapatite coating is in a range of about 0.05% to about 5.5%, about 0.2% to about 5.5%, about 1% to about 5.5%, about 1.5% to about 5.5%, about 2.5% to about 5.5% or about 3% to about 5.5%. In some embodiments, the at least one dopant metal's atomic percentage is about 0.2%, about 0.6%, about 0.7%, about 0.9%, about 1.4%, about 1.8%, about 2.1%, about 3.6%, about 3.7%, about 4%, or about 5.1% in the doped portion.

In some embodiments, the doped hydroxyapatite coating comprises at least two dopant metals. The at least two metals may be present in the doped portion of the doped hydroxyapatite coating in an amount of at least 0.1% by weight of the doped portion for the first dopant metal and an amount of at least 0.1% by weight of the doped portion of a second dopant metal. In some embodiments, the first dopant is present in the doped portion in an amount of about 0.1%, about 0.5%, about 1%, about 1.5%, about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5%, about 10%, about 10.5%, about 11%, about 11.5%, about 12%, about 12.5%, about 13%, about 13.5%, about 14%, about 14.5%, about 15%, about 15.5%, about 16%, about 16.5%, about 17%, about 17.5%, about 18%, about 18.5%, about 19%, about 19.5%, about 20%, about 20.5%, about 21%, about 21.5%, about 22%, about 22.5%, about 23%, about 23.5%, about 24%, about 24.5%, about 25%, about 25.5%, about 26%, about 27%, about 28%, about 29%, or about 30% by weight of the doped portion. In some embodiments, the second dopant metal is present in the doped portion in an amount of about 0.1%, about 0.5%, about 1%, about 1.5%, about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5%, about 10%, about 10.5%, about 11%, about 11.5%, about 12%, about 12.5%, about 13%, about 13.5%, about 14%, about 14.5%, about 15%, about 15.5%, about 16%, about 16.5%, about 17%, about 17.5%, about 18%, about 18.5%, about 19%, about 19.5%, about 20%, about 20.5%, about 21%, about 21.5%, about 22%, about 22.5%, about 23%, about 23.5%, about 24%, about 24.5%, about 25%, about 25.5%, about 26%, about 27%, about 28%, about 29%, or about 30% by weight of the doped portion. In some embodiments, the doped portion comprises at least two dopant metals ranging from about 0.5% to about 30% by weight of the doped portion for a first dopant metal and about 0.5% to about 30% by weight for a second dopant metal. In some embodiments, the first dopant metal is present in the doped portion in an amount of about 2% to about 6% by weight of the coating, about 3% to about 5% by weight of the doped portion, about 3% to about 4% by weight of the coating, about 3.5% to about 4.5% by weight of the doped portion, or about 3.5% to about 4% by weight; and the second dopant metal is present in the doped portion in an amount of about 2% to about 6% by weight of the doped portion, about 3% to about 5% by weight of the doped portion, about 4.5% to about 5.5% by weight of the doped portion, or about 4.5% to about 5% by weight of the doped portion. In some embodiments, the first dopant is present in the doped portion in an amount of about 3.7% by weight of the doped portion and the second dopant is present in doped portion in an amount of about 4.7% by weight of the doped portion.

In some embodiments, the doped hydroxyapatite coating comprises at least two dopant metals present at a particular atomic percentage. In some embodiment, the atomic percentage of a first dopant metal in the doped portion of the doped hydroxyapatite coating is in a range of about 0.05% to about 5.5%, about 0.05% to about 5.2%, about 0.5% to about 5.2%, about 1% to about 4%, about 0.5% to about 2%, about 0.10% to about 1%, about 0.1% to about 0.5%, about 0.5% to about 1%, about 1% to about 2%, about 2% to about 4%, or about 3.5% to about 4%. In some embodiments, the atomic percentage of a second dopant metal in the doped portion is in a range of about 0.05% to about 5.5%, about 0.05% to about 5.2%, about 0.5% to about 5.2%, about 1% to about 4%, about 0.5% to about 2%, about 0.1% to about 1%, about 0.1% to about 0.5%, about 0.5% to about 1%, about 1% to about 2%, about 2% to about 4%, or about 3.5% to about 4%. In some embodiments, the first dopant metal's atomic percentage is about 1.4%, and the second dopant metal's atomic percentage is about 3.7%.

The crystal structure of the doped hydroxyapatite coating can be characterized using X-ray spectroscopy, such as X-ray powder diffraction spectroscopy. The doped hydroxyapatite coatings exhibit several characteristic 2θ diffraction angles when characterized by X-ray powder diffraction. The numbers shown in parenthesis are the Miller indices associated with each peak. Miller indices are a notation system in crystallography. Miller indices describe crystal planes in the format "(hkl)," where each of h, k, and 1 relate to the x-, y-, and z-axis, respectively, in a unit cell. As an example an "(002)" value represents a specific plane of the crystal structure, in particular one that would be parallel to the x axis ("0"), parallel to the y axis ("0"), and intercept the Z-axis at a specific point (0, 0, ½), in this case leading to a value of 2, providing a Miller index of (002). The X-ray pattern using Cu Kα radiation of the doped hydroxyapatite coatings may exhibit 2θ diffraction angles including about 26 ± 2° (002), about 28 ± 2° (102), about 32 ± 2° (112), about 50 ± 2° (213), and about 53 ± 2° (004) or about 26 ± 0.5° (002), about 28 ± 0.5° (102), about 32 ± 0.5° (112), about 50 ± 0.5° (213), and about 53 ± 0.5° (004). The X-ray pattern of the doped hydroxyapatite coatings may exhibit 2θ diffraction angles including about 26 ± 1° (002), about 28 ± 1° (102), about 32 ± 1° (112), about 50 ± 1° (213), and about 53 ± 1° (004). The X-ray patterns of the doped hydroxyapatite coatings may exhibit 2θ diffraction angles including about 25.58 ± 0.1°, about 28.13 ± 0.1°, about 31.75 ± 0.1°, 32.17 ± 0.1°, about 49 ± 0.1°, and about 53 ± 0.1°. It is to be understood that the diffraction angles recited herein may be systematically shifted due to variations in instrumentation. In some embodiments, the doped hydroxyapatite coating, when subjected to XRD, produces a (002) XRD peak and a (112) XRD peak. In illustrative embodiments, the (002) XRD peak and the (112) XRD peak are substantially similar to the XRD peaks of the hydroxyapatite coating that does not comprise a dopant metal.

In illustrative embodiments, the process of incorporating the dopant metal may increase the percent crystallinity of the coating. In some embodiments, the percent crystallinity of a hydroxyapatite coating may be less than about 85% or about 83%. In some embodiments, the percent crystallinity of a hydroxyapatite coating is about 50% to about 85%. In some embodiments, the percent crystallinity of a doped hydroxyapatite coating may be at least about 85%, at least about 90%, or at least about 95%. In some embodiments, the percent crystallinity of a doped hydroxyapatite coating is about 85% to about 99%, about 90% to about 99%, or about 95% to about 99%. In some embodiments, the percent crystallinity of a doped hydroxyapatite coating is about 85%, about 86%, about 87%, about 88%, about 89%, about 90%, about 91%, about 92%, about 93%, about 94%, about 95%, about 96%, about 97%, about 98%, or about 99%.

In illustrative embodiments, the crystallinity improves by at least about 5%, at least about 15%, or at least about 25% because of the process of incorporating the dopant metal. In some embodiments, the crystallinity improves in a range of about 5% to about 100%, about 5% to about 80%, about 5% to about 75%, about 10% to about 75%, about 10% to about 50%, or about 15% to about 50%. Illustratively, an improvement in crystallinity from about 83% to about 97% represents a percent improvement of about 17%.

In some embodiments, the doped hydroxyapatite coating as described above provides a functional advantage when compared to the undoped hydroxyapatite coating. In some embodiments, the dopant metal improves osseointegration, provides infection prophylaxis, is antibacterial, is fluorescent, or a combination thereof when compared to the undoped hydroxyapatite coating.

Illustratively, a doped hydroxyapatite coating comprising zinc, for example, can demonstrate improved antibacterial properties compared to an undoped hydroxyapatite coating. In some embodiments, about 10-fold, about 100-fold, or about 1,000-fold less bacteria grow in a solution in the presence of the doped hydroxyapatite coating compared to the undoped hydroxyapatite coating. In some embodiments, the improvement is at least about 10-fold, at least about 100-fold, or at least about 1,000-fold relative to the undoped hydroxyapatite coating.

In some embodiments, a process is described for forming a coating as described herein. The process comprises contacting a hydroxyapatite-coated substrate with an aqueous solution comprising a dopant metal ion and washing the doped hydroxyapatite-coated substrate to form a doped hydroxyapatite coating. In illustrative embodiments, the XRD pattern of the hydroxyapatite coating before the step of contacting is substantially the same as the XRD pattern after the step of washing such that new crystalline phases are not identified. In some embodiments, the doped hydroxyapatite coating, when exposed to XRD, produces a (002) XRD peak and a (112) XRD peak. In some embodiments, the process produces a doped hydroxyapatite coating where the concentration of the dopant metal is anisotropic in the doped hydroxyapatite coating.

In some embodiments, the hydroxyapatite coating is applied to the substrate by a plasma spray. In some embodiments, a titanium substrate is coated with hydroxyapatite using plasma sprayed hydroxyapatite.

In illustrative embodiments, the process includes a step of contacting. In some embodiments, the contacting step comprises contacting the hydroxyapatite-coated substrate with an aqueous solution comprising a dopant metal. In some embodiments, the contacting step occurs when the hydroxyapatite coating is exposed to the aqueous solution comprising the dopant metal. In some embodiments, the hydroxyapatite-coated substrate is positioned in a container and the aqueous solution is added to the container. In some embodiments, the aqueous solution covers a part or all of the hydroxyapatite-coated substrate. Illustratively, the hydroxyapatite-coated substrate may be submerged in the aqueous solution.

In some embodiments, the aqueous solution comprises at least one dopant metal ion formed by dissolving a metal salt in water. In some embodiments, the aqueous solution comprises a metal salt or a mixture of metal salts. In some embodiments, the melt salt comprises zinc, gallium, copper, europium, silver, or a combination thereof. In some embodiments, the aqueous solution comprises at least one dopant metal ion. The at least one dopant metal ion is present in the aqueous solution in an amount of at least about 0.01 mM. In some embodiments, the at least one dopant metal ion is present in the aqueous solution in a concentration of about 0.01 mM to about 50 mM, about 0.01 mM to about 30 mM, or about 0.01 mM to about 20 mM.

In some embodiments, the contacting step is performed with at least one aqueous solution and the hydroxyapatite-coated substrate. In some embodiments, the contact step includes a first step of contacting with a first aqueous solution and a second step of contacting with a second aqueous solution. In some embodiments, a step of washing occurs between the first aqueous solution contacting the hydroxyapatite-coated substrate and a second aqueous solution contacting the hydroxyapatite-coated substrate. In some embodiments, a first aqueous solution and a second aqueous solution contact the hydroxyapatite-coated substrate consecutively or concurrently. In some embodiments, the first aqueous solution and the second aqueous solution each include a different metal ion. In some embodiments, the first aqueous solution and the second aqueous solution include at least one common metal ion.

In some embodiments, the step of exchanging is performed by contacting the hydroxyapatite-coated substrate with an aqueous solution comprising a dopant metal ion. Illustratively, the step of exchanging replaces lattice positions in the hydroxyapatite coating with the dopant metal.

In some embodiments, the process of forming a coating comprises contacting a hydroxyapatite-coated substrate with a first aqueous solution comprising a first dopant metal, washing the doped hydroxyapatite-coated substrate, contacting the doped hydroxyapatite-coated substrate with a second aqueous solution, and washing the doped hydroxyapatite-coated substrate of the second aqueous solution. In some embodiments, a drying step is included between the first washing step the second contacting step with a second aqueous solution. In some embodiments, the drying step is performed at about 60 °C for about 2 hours.

In some embodiments, the aqueous solution has a particular pH. In some embodiments, the aqueous solution has a pH of about 5 to about 9. In some embodiments, the aqueous solution has a pH of about 5, about 5.5, about 6, about 6.5, about 7, about 7.5, about 8, or about 8.5. Illustratively, the pH of the solution may contribute to the ability of the locally dissolved hydroxyapatite to recrystallize onto the surface of the substrate. In some illustrative embodiments, the pH may differ between the dopant metals.

In some embodiments, the contacting step is performed at a temperature of at least 25 °C or at least 60 °C. In some embodiments, the contacting step is performed at a temperature of about 20 °C, about 21 °C, about 22 °C, about 23 °C, about 24 °C, about 25 °C, about 26 °C, about 27 °C, about 28 °C, about 50 °C, about 51 °C, about 52 °C, about 53 °C, about 54 °C, about 55 °C , about 56 °C, about 57 °C, about 58 °C, about 59 °C, about 60 °C, about 61 °C, about 62 °C, about 63 °C, about 64 °C, about 65 °C, about 66 °C, about 67 °C , about 68 °C, about 69 °C, about 70 °C, about 71 °C, about 72 °C, about 73 °C, about 74 °C, about 75 °C, about 85 °C, about 90 °C, about 95 °C, about 100 °C, about 105 °C, about 110 °C, about 115 °C, about 116 °C, about 117 °C, about 118 °C, about 119 °C, about 120 °C, about 121 °C, about 122 °C, about 123 °C, about 124 °C, about 125 °C, about 130 °C, about 135 °C, about 140 °C, about 145 °C, about 146 °C, about 147 °C, about 148 °C, about 149 °C, about 150 °C, about 151 °C, about 152 °C, about 153 °C, about 154 °C, about 155 °C, about 160 °C, about 165 °C, about 170 °C, or about 175 °C. In some embodiments, the contacting step is performed at a temperature of about 25 °C to about 175 °C, about 25 °C to about 60 °C, about 60 °C to about 120 °C, about 120 °C to about 150 °C, about 150 °C to about 175 °C, about 23 °C to about 27 °C, about 57 °C to about 62 °C, about 117 °C to about 123 °C, about 147 °C to about 152 °C, or about 172 °C to about 177 °C.

In some embodiments, a first contacting step occurs at about a first temperature and a second contacting step occurs at about a second temperature. The first and second temperature may be about the same temperature, or the first and second temperature may be different temperatures. The difference between the first and second temperatures may be slight or it may be significant. In some embodiments, the first contacting step is performed at a temperature of about 150 °C, and the second contacting step is performed at a temperature of about 120 °C. In some embodiments, the first contacting step is performed at a temperature of about 120 °C, and the second contacting step is performed at a temperature of about 150 °C.

In some embodiments, the contacting step is performed for a time of at least 25 minutes. In some embodiments, the contacting step is performed for a time of about 0.5, about 1, about 1.5, about 2, about 2.5, about 3, about 3.5, about 4, about 4.5, about 5, about 5.5, about 6, about 6.5, about 7, about 8, about 9, about 10, about 11, about 12, about 13, about 14, about 15, about 16, about 17, about 18, about 19, about 20, about 21, about 22, about 23, about 23.5, about 24, about 24.5, about 25, about 26, about 27, about 28, about 29, about 30, about 36, about 42, about 48, about 54, about 60, about 66, about 72, about 78, or about 80 hours. In some embodiments, the contacting step is performed for a time of about 30 minutes (i.e., about 0.5 hours) to about 72 hours. In some embodiments, the contacting step is performed for at time of about 0.5 hour to about 1 hour, about 1 hour to about 2 hours, about 2 hours to about 4 hours, about 2 hours to about 3 hours, about 3 hours to about 4 hours, about 4 hours to about 6 hours, about 5 hours to about 6 hours, about 6 hours to about 24 hours, about 23 hours to about 24 hours, about 24 hours to about 72 hours, or about 71 hours to about 72 hours. In some embodiments, the contacting step is performed for a time of about 1 hour. In some embodiments, the contacting step is performed for a time of about 2 hours. In some embodiments, the contacting step is performed for about 4 hours. In some embodiments, the contacting step is performed for a time of about 6 hours. In some embodiments, the contacting step is performed for a time of about 24 hours. In some embodiments, the contacting step is performed for about 48 hours. In some embodiments, the contacting step is performed for about 72 hours.

In some embodiments, a first contacting step is performed for a time of about 0.5 hour to about 72 hours, and a second contacting step is performed for a time of about 0.5 hour to about 72 hours. In some embodiments, the first contacting step and the second contacting step are performed for a similar amount of time. In some embodiments, the first contacting step is performed for a longer time than the second contacting step. In some embodiments, the first contacting step is performed for a shorter time than the second contacting step. In some embodiments, the first contacting step is performed for about 24 hours and the second contacting step is performed for about 4 hours. In some embodiments, the first contacting step is performed for about 4 hours and the second contacting step is performed for about 24 hours.

In some embodiments, the contacting step is performed with the hydroxyapatite-coated substrate and the aqueous solution sealed in a container. In some embodiments, this container is tightly sealed. In some embodiments, the contacting step is performed in a temperature-controlled oven.

In some embodiments, the doped hydroxyapatite-coated substrate was analyzed using XRD. In some embodiments, the XRD pattern of the hydroxyapatite coating before the step of contacting is substantially the same as the XRD pattern after the step of washing. In some embodiments, the doped hydroxyapatite coating, when exposed to XRD, produces a (002) XRD peak and a (112) XRD peak.

In some embodiments, the doped hydroxyapatite coating comprises zinc in an amount of about 7% by weight to about 11% by weight in the doped portion. In some embodiments, the doped hydroxyapatite coating comprises zinc in an amount of about 9% by weight to about 10% by weight in the doped portion. In some embodiments, the aqueous solution used for incorporating zinc into the doped portion had a pH about 7 to about 7.6, preferably about 7.1 to about 7.5.

In some embodiments, the doped hydroxyapatite coating comprises gallium in an amount of about 1% by weight to about 4% by weight in the doped portion. In some embodiments, the doped hydroxyapatite coating comprises gallium in an amount of about 2% by weight to about 3% by weight in the doped portion. In some embodiments, the aqueous solution used for incorporating gallium into the doped portion had a pH about 8 to about 8.5, preferably about 8.2 to about 8.4.

In some embodiments, the doped hydroxyapatite coating comprises copper in an amount of about 0.1% by weight to about 3% by weight in the doped portion. In some embodiments, the doped hydroxyapatite coating comprises copper in an amount of about 0.25% by weight to about 1% by weight in the doped portion. In some embodiments, the aqueous solution used for incorporating copper into the doped portion had a pH about 5.5 to about 6, preferably about 5.6 to about 5.8.

In some embodiments, the doped hydroxyapatite coating comprises europium in an amount of about 10% by weight to about 25% by weight in the doped portion. In some embodiments, the doped hydroxyapatite coating comprises europium in an amount of about 12% by weight to about 13% by weight in the doped portion. In some embodiments, the doped hydroxyapatite coating comprises europium in an amount of about 22% by weight to about 23% by weight in the doped portion. In some embodiments, the aqueous solution used for incorporating europium into the doped portion had a pH about 6 to about 7.5, preferably about 6.5 to about 7.

In some embodiments, the doped hydroxyapatite coating comprises silver in an amount of about 1.5% by weight to about 25% by weight in the doped portion. In some embodiments, the doped hydroxyapatite coating comprises silver in an amount of about 7% by weight to about 18% by weight in the doped portion. In some embodiments, the doped hydroxyapatite coating comprises silver in an amount of about 19% by weight to about 20% by weight in the doped portion. In some embodiments, the doped hydroxyapatite coating comprises silver in an amount of about 2% by weight to about 3% by weight in the doped portion. In some embodiments, the aqueous solution used for incorporating silver into the doped portion had a pH about 5 to about 6, preferably about 5.4 to about 5.9.

In some embodiments, the doped hydroxyapatite coating comprises zinc and copper in an amount of about 3% by weight to about 6% by weight for the copper and about 2% by weight to about 5% by weight for the zinc in the doped portion. In some embodiments, the doped hydroxyapatite coating comprises zinc and copper in an amount of about 4% by weight to about 5% by weight for the copper and about 3% by weight to about 4% by weight for the zinc in the doped portion.

In some embodiments, the doped hydroxyapatite coating comprises zinc having an atomic percentage of about 2% to about 5% in the doped portion. In some embodiments, the doped hydroxyapatite coating comprises zinc having an atomic percentage of about 3% to about 4% in the doped portion.

In some embodiments, the doped hydroxyapatite coating comprises gallium in an atomic percentage of about 0.1% to about 3% in the doped portion. In some embodiments, the doped hydroxyapatite coating comprises gallium having an atomic percentage of about 0.75% to about 1% in the doped portion.

In some embodiments, the doped hydroxyapatite coating comprises copper having an atomic percentage of about 0.05% to about 1% in the doped portion. In some embodiments, the doped hydroxyapatite coating comprises copper having an atomic percentage of about 0.6% to about 0.1% in the doped portion. In some embodiments, the doped hydroxyapatite coating comprises copper having an atomic percentage of about 0.15% to about 0.2% in the doped portion.

In some embodiments, the doped hydroxyapatite coating comprises europium having an atomic percentage of about 1.5% to about 5% in the doped portion. In some embodiments, the doped hydroxyapatite coating comprises europium having an atomic percentage of about 2% to about 3% in the doped portion. In some embodiments, the doped hydroxyapatite coating comprises europium having an atomic percentage of about 3% to about 4% in the doped portion.

In some embodiments, the doped hydroxyapatite coating comprises silver having an atomic percentage of about 0.1% to about 6.5% in the doped portion. In some embodiments, the doped hydroxyapatite coating comprises silver having an atomic percentage of about 1.5% to about 2% in the doped portion. In some embodiments, the doped hydroxyapatite coating comprises silver having an atomic percentage of about 5% to about 6% in the doped portion. In some embodiments, the doped hydroxyapatite coating comprises silver having an atomic percentage of about 0.5% to about 0.7% in the doped portion.

In some embodiments, the doped hydroxyapatite coating comprises at least two dopant metals wherein a first dopant is present having an atomic percentage of about 0.5% to about 5% and a second dopant present having an atomic percentage of about 0.5% in the doped portion. In some embodiments, the doped hydroxyapatite coating comprises zinc having an atomic percentage of about 1% to about 2% and copper having an atomic percentage of about 3% to about 4% in the doped portion. In some embodiments, the doped hydroxyapatite coating comprises zinc having an atomic percentage of about 1.3% to about 1.4% and copper having an atomic percentage of about 3.7% to about 3.8% in the doped portion. In some embodiments, a zinc incorporated hydroxyapatite-coated substrate is formed by contacting a hydroxyapatite-coated substrate with a Zinc solution. In some embodiments, the contacting step is performed at about 150 °C for about 72 hours. In some embodiments, the contacting step is performed in a tightly sealed container. In some embodiments, a zinc incorporated hydroxyapatite-coated substrate is formed after the contacting step. In some embodiments, a washing step is performed on the zinc incorporated hydroxyapatite-coated substrate with an aqueous solution. In some embodiments, the aqueous solution is deionized water. In some embodiments, a drying step is performed on the zinc incorporated hydroxyapatite-coated substrate. In some embodiments, the drying step is performed at a temperature of about 60 °C for about 2 hours. In some embodiments, the zinc incorporated hydroxyapatite-coated substrate is analyzed using EDX and XRD, which provide patterns. In some embodiments, the XRD patterns of the hydroxyapatite-coated substrate and the zinc incorporated hydroxyapatite-coated substrate are substantially similar.

In some embodiments, a gallium incorporated hydroxyapatite-coated substrate is formed by contacting a hydroxyapatite-coated substrate with a Gallium solution. In some embodiments, the contacting step is performed at about 150 °C for about 72 hours. In some embodiments, the contacting step is performed in a tightly sealed container. In some embodiments, the gallium incorporated hydroxyapatite-coated substrate is formed after the contacting step. In some embodiments, a washing step is performed on the gallium incorporated hydroxyapatite-coated substrate with an aqueous solution. In some embodiments, the aqueous solution is deionized water. In some embodiments, a drying step is performed on the gallium incorporated hydroxyapatite-coated substrate. In some embodiments, the drying step is performed at a temperature of about 60 °C for about 2 hours. In some embodiments, the gallium incorporated hydroxyapatite-coated substrate is analyzed using EDX and XRD, which provide patterns. In some embodiments, the XRD patterns of the hydroxyapatite-coated substrate and the gallium incorporated hydroxyapatite-coated substrate are substantially similar. In some embodiments, the percentage by weight of calcium in the gallium treated coating is less than the percentage by weight of calcium in the untreated coating.

In some embodiments, a copper incorporated hydroxyapatite-coated substrate is formed by contacting a hydroxyapatite-coated substrate with a copper solution. In some embodiments, the contacting step is performed at about 120 °C for about 4 hours. In some embodiments, the contacting step is performed at about 60 °C for about 24 hours. In some embodiments, the contacting step is performed in a tightly sealed container. In some embodiments, the copper incorporated hydroxyapatite-coated substrate is formed after the contacting step. In some embodiments, a washing step is performed on the copper incorporated hydroxyapatite-coated substrate with an aqueous solution. In some embodiments, the aqueous solution is deionized water. In some embodiments, a drying step is performed on the copper incorporated hydroxyapatite-coated substrate. In some embodiments, the drying step is performed at a temperature of about 60 °C for about 2 hours. In some embodiments, the copper incorporated hydroxyapatite-coated substrate is analyzed using EDX and XRD, which provide patterns. In some embodiments, the XRD patterns of the hydroxyapatite-coated substrate and the copper incorporated hydroxyapatite-coated substrate are substantially similar. In some embodiments, the percentage by weight of calcium in the copper treated coating is less than the percentage by weight of calcium in the untreated coating.

In some embodiments, a europium incorporated hydroxyapatite-coated substrate is formed by contacting a hydroxyapatite-coated substrate with a europium solution. In some embodiments, the contacting step is performed at about 120 °C for about 2 hours. In some embodiments, the contacting step is performed at about 60 °C for about 24 hours. In some embodiments, the contacting step is performed in a tightly sealed container. In some embodiments, the europium incorporated hydroxyapatite-coated substrate is formed after the contacting step. In some embodiments, a washing step is performed on the europium incorporated hydroxyapatite-coated substrate with an aqueous solution. In some embodiments, the aqueous solution is deionized water. In some embodiments, a drying step is performed on the europium incorporated hydroxyapatite-coated substrate. In some embodiments, the drying step is performed at a temperature of about 60 °C for about 2 hours. In some embodiments, the europium incorporated hydroxyapatite-coated substrate is analyzed using EDX and XRD, which provide patterns. In some embodiments, the XRD patterns of the hydroxyapatite-coated substrate and the europium incorporated hydroxyapatite-coated substrate are substantially similar. In some embodiments, the percentage by weight of calcium in the europium treated coating is less than the percentage by weight of calcium in the untreated coating.

In some embodiments, a silver incorporated hydroxyapatite-coated substrate is formed by contacting a hydroxyapatite-coated substrate with a silver solution. In some embodiments, the contacting step is performed at about 60 °C for about 24 hours. In some embodiments, the contacting step is performed at about 120 °C for about 4 hours. In some embodiments, the contacting step is performed at about 25 °C for about 24 hours. In some embodiments, the contacting step is performed in a tightly sealed container. In some embodiments, the silver incorporated hydroxyapatite-coated substrate is formed after the contacting step. In some embodiments, a washing step is performed on the silver incorporated hydroxyapatite-coated substrate with an aqueous solution. In some embodiments, the aqueous solution is deionized water. In some embodiments, a drying step is performed on the silver incorporated hydroxyapatite-coated substrate. In some embodiments, the drying step is performed at a temperature of about 60 °C for about 2 hours. In some embodiments, the silver incorporated hydroxyapatite-coated substrate is analyzed using EDX and XRD, which provide patterns. In some embodiments, the XRD patterns of the hydroxyapatite-coated substrate and the silver incorporated hydroxyapatite-coated substrate are substantially similar. In some embodiments, the percentage by weight of calcium in the silver treated coating is less than the percentage by weight of calcium in the untreated coating.

In some embodiments, a zinc-copper incorporated hydroxyapatite-coated substrate is formed by first contacting a hydroxyapatite-coated substrate with a zinc solution. In some embodiments, the contacting step is performed at about 150 °C for about 24 hours. In some embodiments, the first contacting step is performed in a tightly sealed container. In some embodiments, a zinc incorporated hydroxyapatite-coated substrate is formed after the first contacting step. In some embodiments, a washing step is performed to remove the zinc solution. In some embodiments, the washing step is performed with deionized water. In some embodiments, a first drying step is performed at about 60 °C for about 2 hours. In some embodiments, no first drying step is performed. In some embodiments, a second contacting step is performed using a copper solution. In some embodiments, the second contacting step is performed at about 120 °C for about 4 hours. In some embodiments, the second contacting step is performed in a tightly sealed container. In some embodiments, a zinc-copper incorporated hydroxyapatite-coated substrate is formed after the second contacting step. In some embodiments, a second washing step is performed on the zinc-copper incorporated hydroxyapatite-coated substrate with an aqueous solution. In some embodiments, the aqueous solution is deionized water. In some embodiments, a drying step is performed on the zinc-copper incorporated hydroxyapatite-coated substrate. In some embodiments, this is the second drying step. In some embodiments, the drying step is performed at a temperature of about 60 °C for about 2 hours. In some embodiments, the zinc-copper incorporated hydroxyapatite-coated substrate is analyzed using EDX and XRD, which provide patterns. In some embodiments, the XRD patterns of the hydroxyapatite-coated substrate and the zinc-copper incorporated hydroxyapatite-coated substrate are substantially similar. In some embodiments, the percentage by weight of calcium in the zinc-copper treated coating is less than the percentage by weight of calcium in the untreated coating.

In some embodiments, a process is provided for forming a coating. In some embodiments, the process comprises exchanging lattice positions of a hydroxyapatite-coated substrate with a dopant metal to form a doped hydroxyapatite coating, and washing the doped hydroxyapatite coating. In some embodiments, the doped hydroxyapatite coating is analyzed using EDX and XRD. In some embodiments, the XRD pattern of the hydroxyapatite coating before the step of contacting is substantially the same as the XRD pattern after the step of washing. In some embodiments, the XRD patterns of the hydroxyapatite-coated substrate and the doped hydroxyapatite coating are substantially similar.

### Example 1: Preparing a Plasma Sprayed Hydroxyapatite (PSHA) Coating

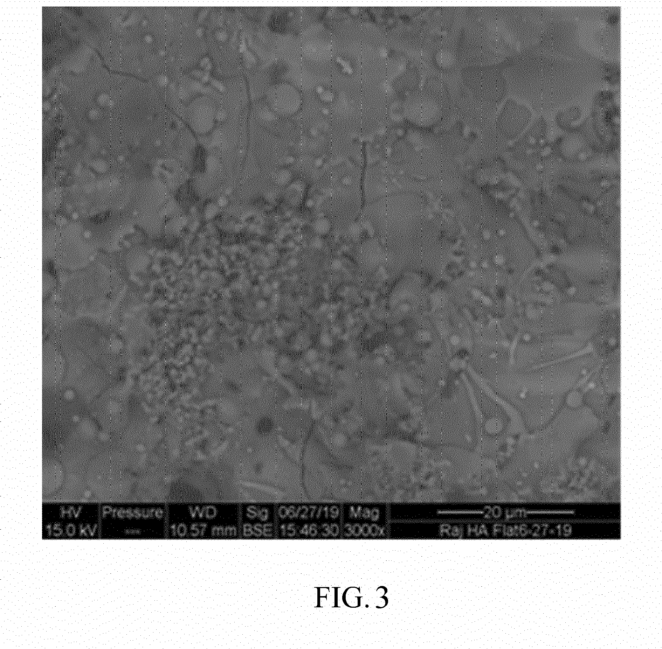

A titanium coupon was plasma spray coated with HA. Briefly, a plasma torch was employed to partially melt and accelerate feedstock HA powders onto the coupon. These semi-molten particles coalesce into a coating upon contacting the coupon. An SEM image of a PSHA-coated coupon is shown in Fig. 3.

**Table 1: Weight percent of the untreated coating**

| **P (wt%)** | **Ca (wt%)** | **O (wt%)** |
|---|---|---|
| 14.8 | 33.0 | 47.2 |

**Table 2: Atomic percentage of the treated coating**

| **P (atomic %)** | **Ca (atomic %)** | **O (atomic %**) |
|---|---|---|
| 10.37 | 17.83 | 63.33 |

### Example 2: Incorporating Zinc (Zn) into a PSHA Coating.

An inorganic phosphate-zinc (PiZn) solution was prepared by combining 9 mL of 1 mM inorganic phosphate (Pi) with 0.275 mL 6N sodium hydroxide (NaOH). Then 1 mL of 100 mM Zn(N0₃)₂ was added to the mixture. The final zinc concentration in the PiZn solution was 10 mM. The pH of the aqueous solution was about 7.3.

A PSHA-coated coupon (1.0 inch in diameter, 0.25 inch in thickness), prepared as described in Example 1, was placed at the bottom of a titanium alloy container (1.0 inch in length, 1.125 inch in inner diameter) with the PSHA coating facing up. 8 mL of the PiZn solution was subsequently added to submerge the PSHA-coated coupon. The solution volume was roughly 70% of the container's inner volume. The container was then sealed with a titanium alloy screw cap. TEFLON tape and an O-ring were used to create a tight seal.

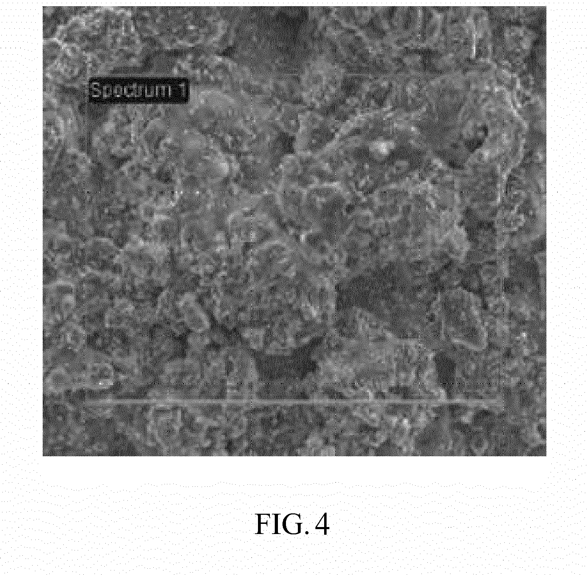

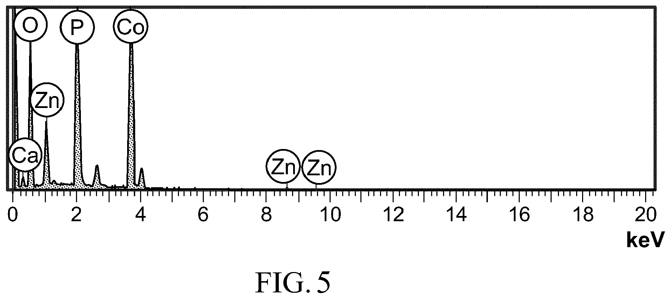

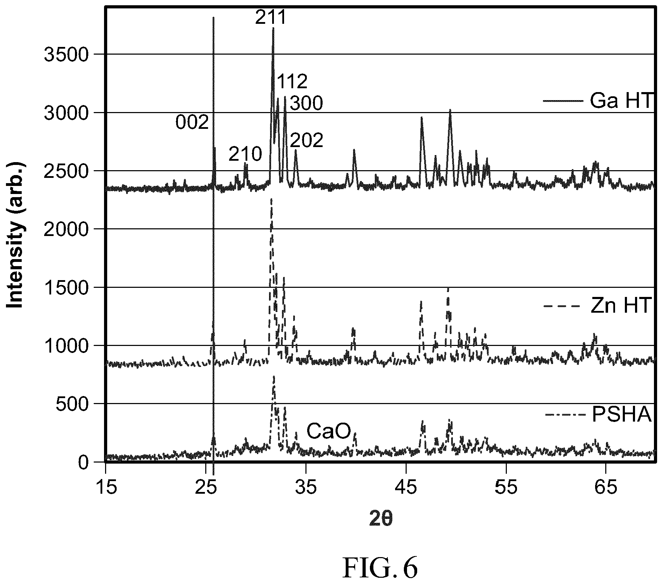

The sealed container was placed in an oven at 150 °C for about 72 hours. The container was removed from the oven and cooled to room temperature. The PiZn treated PSHA coupon was washed with deionized (DI) water. The PiZn treated coupon was then dried at 60 °C for about 2 hours before being analyzed with energy-dispersive X-ray spectroscopy (EDX) and X-ray powder diffraction (XRD). Fig. 4 shows an SEM of the PiZn treated PSHA coating. Fig. 5 shows the EDX pattern of the PiZn treated PSHA coating. Fig. 6 shows the XRD pattern of an untreated PSHA coating (denoted PSHA) and PiZn treated PSHA coating (denoted Zn HT). The XRD shows that the PiZn treated PSHA coating was more crystalline compared to the untreated PSHA coating, and CaO and other calcium phosphate phases could not be detected in the treated coating. Additionally, no new zinc containing phases were detected after treatment. Table 3 shows the chemical composition of the PiZn treated PSHA coating. Table 4 shows the atomic percent of the chemical components in the PiZn treated PSHA coating.

**Table 3: Weight percent of the treated coating**

| **Solution** | **P (wt%)** | **Ca (wt%)** | **O (wt%)** | **Zn (wt%)** |
|---|---|---|---|---|
| PiZn | 14.8 | 32.3 | 43.1 | 9.8 |

**Table 4: Atomic percentage of the treated coating**

| **Solution** | **P (atomic %)** | **Ca (atomic %)** | **O (atomic %)** | **Zn (atomic %**) |
|---|---|---|---|---|
| PiZn | 11.58 | 19.50 | 65.28 | 3.64 |

### Example 3: Incorporating Gallium (Ga) into a PSHA coating.

An inorganic phosphate-gallium (PiGa) solution was prepared by mixing Ga(N03)3 in to 500 mL of 2.51 mM Phosphate stock solution to give 0.39 mM Ga³⁺. After thorough mixing and dissolution the pH was adjusted to 8.3 with micro-liter additions of 6N NaOH

A one-inch PSHA-coated coupon (1.0 inch in length, 1.125 inch in inner diameter) was placed at the bottom of a titanium alloy container with coating facing up. 8 mL of the PiGa solution was subsequently added to submerge the PSHA-coated coupon. The solution volume was roughly 70% of the container's inner volume. The container was then sealed with a titanium alloy screw cap. TEFLON tape and O-ring were used to create a tight seal.

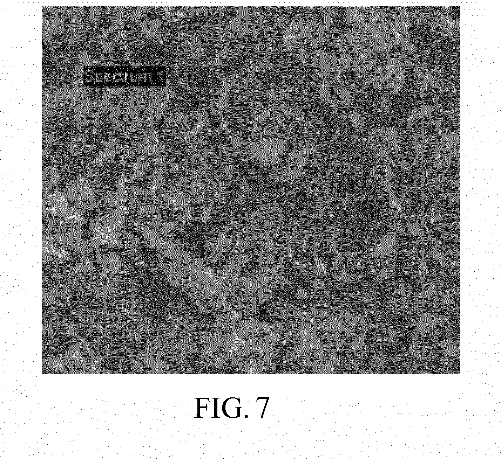

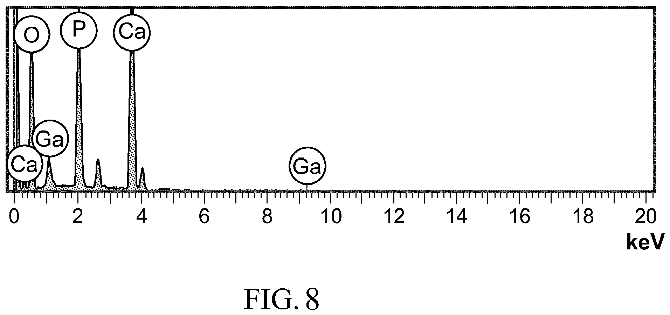

The sealed container was placed in an oven at 150 °C for about 72 hours. The container was removed from the oven and cooled to room temperature. The PiGa treated PSHA coating was washed with DI water. The PiGa treated PSHA coating was then dried at 60 °C for about 2 hours before being analyzed with EDX and XRD. Fig. 7 shows an SEM of the PiGa treated PSHA coating. Fig. 8 shows the EDX pattern of the PiGa treated PSHA coating. Fig. 6, previously referred to in Example 2, shows the XRD pattern of untreated PSHA coating (denoted PSHA) and the PiGa treated PSHA coating (denoted Ga HT). The XRD shows that the PiGa treated PSHA coating is more crystalline than before treatment, and CaO and other calcium phosphate phases could not be detected in the treated coating. Additionally, no new gallium containing phases were detected after the treatment with the PiGa solution. Table 5 shows the chemical composition of the PiGa treated PSHA coating. Table 6 shows the atomic% of the chemical components of the PiGa treated PSHA coating.

**Table 5: Weight percent of the treated coating**

| **Solution** | **P (wt%)** | **Ca (wt%)** | **O (wt%)** | **Ga (wt%)** |
|---|---|---|---|---|
| PiGa | 16.1 | 36.1 | 45.1 | 2.6 |

**Table 6: Atomic percentage of the treated coating**

| **Solution** | **P (atomic %)** | **Ca (atomic %)** | **O (atomic %)** | **Ga (atomic %)** |
|---|---|---|---|---|
| PiGa | 12.17 | 36.14 | 65.90 | 0.86 |

### Example 4: Incorporating Copper (Cu) into a PSHA Coating.

A 0.081 mM copper chloride solution was prepared by mixing 0.0138 grams of cupric chloride with 100 mL of DI water. A 0.1 mM of calcium nitrate solution was made by mixing tetrahydrate, granular calcium nitrate with DI water. Finally, a Cu solution was prepared by mixing 4 mL of the copper chloride solution with 4 mL of the calcium nitrate solution 32 mL of DI water in a 100 mL NALGENE jar. Then, the pH was measured using a pH probe.

Once the Cu solution was prepared, a PSHA-coated coupons, prepared as described in Example 1, was placed at the bottom of a NALGENE jar with the PSHA coating facing up. The solution volume was roughly 40 mL. The container was sealed with an appropriate NALGENE jar lid.

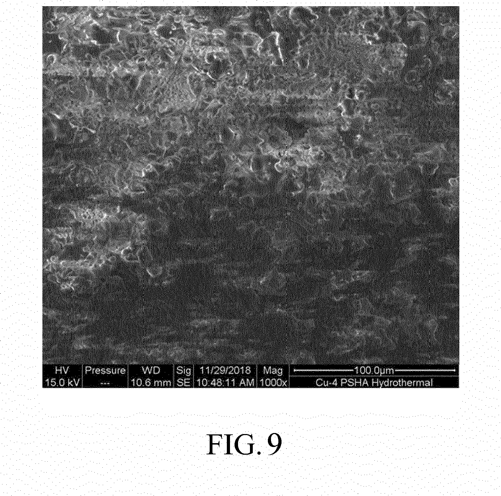

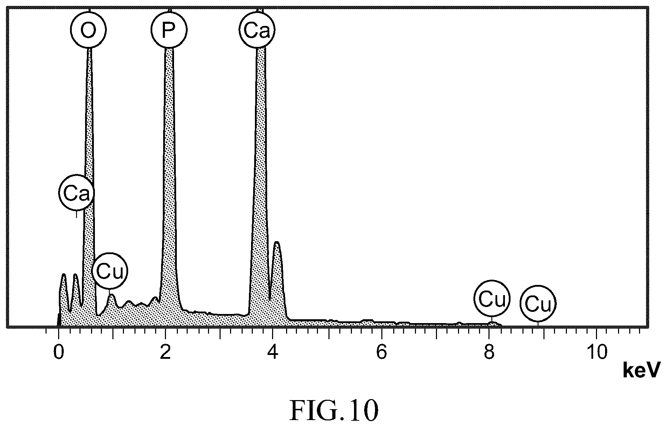

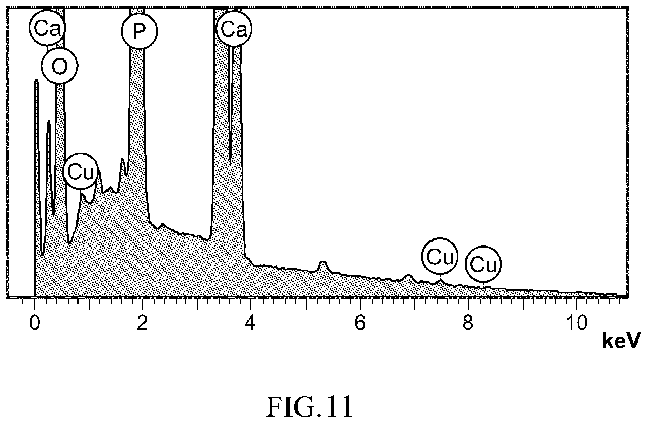

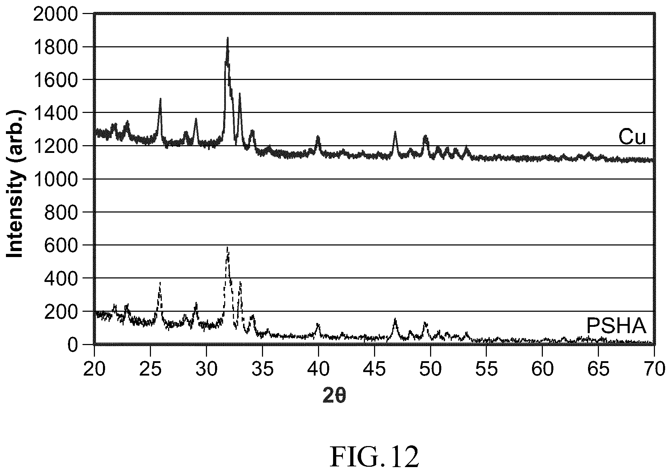

Two NALGENE jars, sometimes called "containers," were prepared as described above in this Example to run two trials (see Table 5). The first container was placed in an oven at 120 °C for 4 hours. The second container was placed in an oven at 60 °C for 24 hours. The containers were removed from the oven and cooled to room temperature. The Cu treated PSHA coatings were washed with DI water. The Cu treated PSHA coatings were then dried at 60 °C for 2 hours before being weighed and analyzed with EDX and XRD. Fig. 9 is an SEM image of the PSHA coating that was treated with Cu solution at 120 °C for 4 hours, showing the PSHA coating incorporating Cu. Fig. 10 is an EDX pattern of the PSHA coating treated with the Cu solution at 120 °C for 4 hours. Fig. 11 is an EDX pattern of the PSHA coating treated with the Cu solution at 60 °C for 24 hours. Fig. 12 shows the XRD pattern of an untreated PSHA coating (denoted PSHA) and a Cu-1 treated PSHA coating (denoted Cu). The XRD shows that the Cu treated PSHA coating was more crystalline than before the treatment. Additionally, no new crystalline phases were detected after treatment. Table 7 lists each trial condition.

**Table 7: Treatment conditions**

| **Coupon** | **Initial pH** | **Conditions** | **Initial Weight** | **Final Weight** |
|---|---|---|---|---|
| Cu-1 | 5.714 | 120 °C for 4h | 7.0657g | 7.0655g |
| Cu-4 | 5.707 | 60 °C for 24h | 7.0664g | 7.0664g |

Table 8 shows the chemical composition of the Cu treated PSHA coupons. Table 9 shows the atomic percent of the chemical components in coupons Cu-1 and Cu-4, analyzed by EDX after treatment (see Table 7 for treatment conditions).

**Table 8: Weight percent of the treated coating**

| **Coupon** | **P (wt%)** | **Ca (wt%)** | **O (wt%)** | **Cu (wt%)** |
|---|---|---|---|---|
| Cu-1 | 15.6 | 34.0 | 48.5 | 1.9 |
| Cu-4 | 15.6 | 34.7 | 49.2 | 0.5 |

**Table 9: Atomic percentage of the treated coating**

| **Solution** | **P (atomic %)** | **Ca (atomic %)** | **O (atomic %)** | **Cu (atomic %)** |
|---|---|---|---|---|
| Cu-1 | 11.42 | 19.20 | 68.71 | 0.68 |
| Cu-4 | 11.31 | 19.46 | 69.04 | 0.19 |

### Example 5: Incorporating Europium (Eu) into a PSHA Coating.

A solution of 38 mL of europium chloride (0.001 N) and of 2 mL Pi stock solution was prepared in a 100 mL NALGENE jar to create a Eu solution. Once the pH was approximately measured to be roughly 6, the solution was titrated with roughly 125 µL of a NaOH. The final pH stabilized around 7.

Once the Eu solution was prepared, a PSHA-coated coupon was placed at the bottom of a NALGENE jar with the PSHA coating facing up. The Eu solution volume was approximately 40 mL. The containers were each sealed with an appropriate NALGENE jar lid.

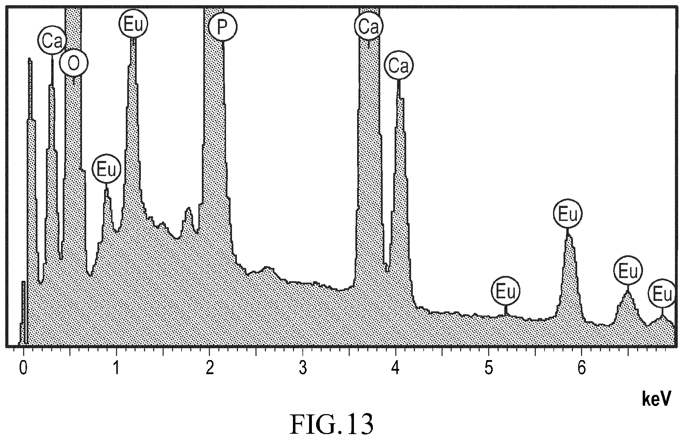

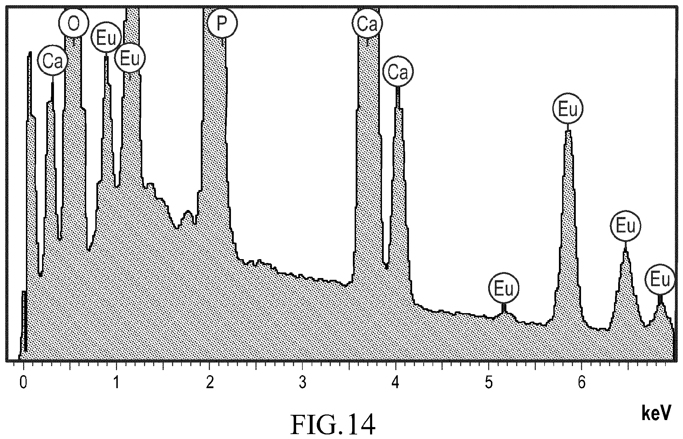

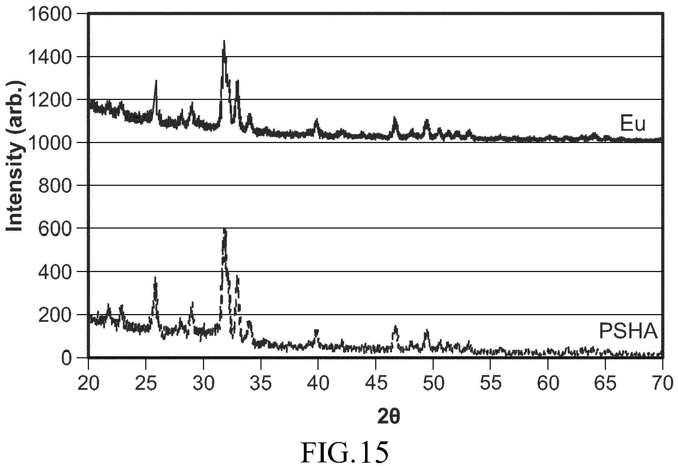

Two containers were prepared as described above in this Example 5 to run two trials (See Table 10). The first container was placed in an oven at 120 °C for 2 hours. The second container was placed in an oven at 60 °C for 24 hours. Each container was removed from the oven and cooled to room temperature. The Eu treated PSHA coatings were washed with DI water. The Eu treated PSHA coatings were then dried at 60 °C for 2 hours before being weighed and analyzed with EDX and XRD. Fig. 13 is an image showing an EDX pattern of the PSHA coating treated with the Eu solution at 120 °C for 2 hours. Fig. 14 is an image of an EDX pattern of the PSHA coating treated with the Eu solution at 60 °C for 24 hours. Fig. 15 is a graph showing an XRD pattern of untreated PSHA coating (denoted PSHA) and the PSHA coating treated with the Eu solution at 60 °C for 24 hours (denoted Eu-PSHA). The XRD pattern in Fig. 15 shows that the Eu treated PSHA coating is more crystalline than before treatment and new calcium phosphate phases could not be detected in the coating. Additionally, europium crystalline phases were not detected after the treatment. Table 10 lists each trial condition. Table 11 shows the chemical composition of the Eu treated PSHA coatings. Table 12 shows the atomic percent of the chemical components within each Eu treated PSHA coatings (see Table 10). The information for Tables 11 and 12 were generated from an EDX analysis.

**Table 10: Treatment conditions**

| **Coupon** | **Initial pH** | **Final pH** | **Conditions** | **Initial Weight** | **Final Weight** |
|---|---|---|---|---|---|
| Eu-3 | 6.030 | 7.089 | 120 °C for 2h | 7.09025 g | 7.08986 g |
| Eu-4 | 6.130 | 7.071 | 60 °C for 24h | 7.06442 g | 7.06380 g |

**Table 11: Weight percent of the treated coating**

| **Coupon** | **P (wt%)** | **Ca (wt%)** | **O (wt%)** | **Eu (wt%)** |
|---|---|---|---|---|
| Eu-3 | 14.7 | 28.2 | 44.3 | 12.8 |
| Eu-4 | 13.7 | 21.5 | 42.1 | 22.7 |

**Table 12: Atomic percentage of the treated coating**

| **Solution** | **P (atomic %)** | **Ca (atomic %)** | **O (atomic %)** | **Eu (atomic %)** |
|---|---|---|---|---|
| Eu-3 | 11.75 | 17.47 | 68.70 | 2.08 |
| Eu-4 | 11.80 | 14.28 | 69.96 | 3.96 |

### Example 6: Incorporating Silver (Ag) into a PSHA Coating.

A 0.1 mM calcium nitrate solution was prepared by mixing approximately 0.0236 grams of tetrahydrate granular calcium nitrate were mixed with 100 mL of DI water. Then to prepare the final Silver solution, 2 mL of 0.1 mM calcium nitrate solution, 6 mL of 0.1 M silver nitrate, and 32 mL of DI water were mixed together in a NALGENE jar. The pH was then measured using a pH probe.

Once the Ag solution was prepared, a PSHA-coated coupon was placed at the bottom of a 100 mL NALGENE jar with the PSHA coating facing up. The solution volume was roughly approximately 40 mL prior to adding the PSHA-coated coupon. The container was then sealed with an appropriate NALGENE jar cap.

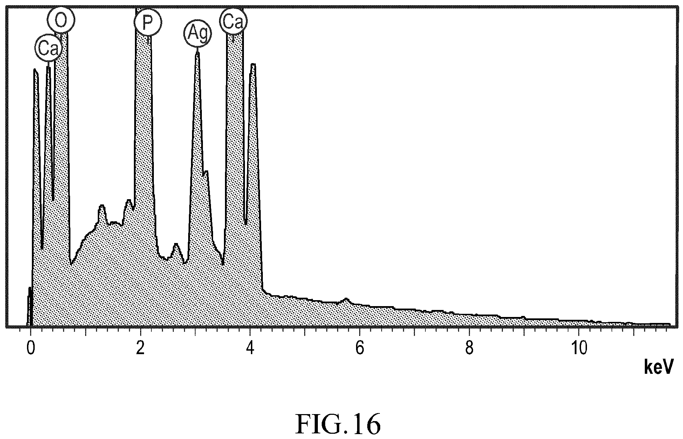

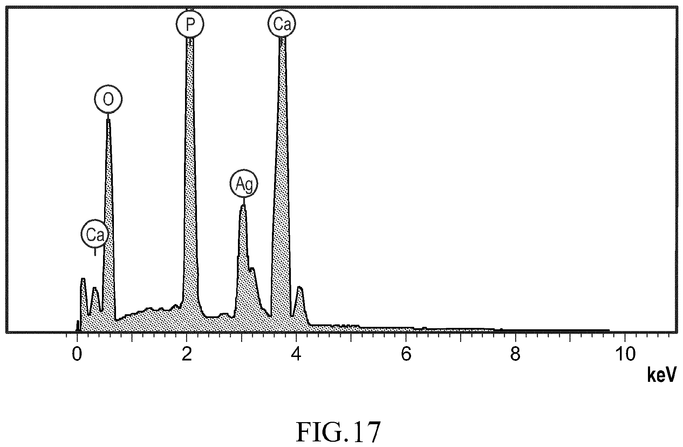

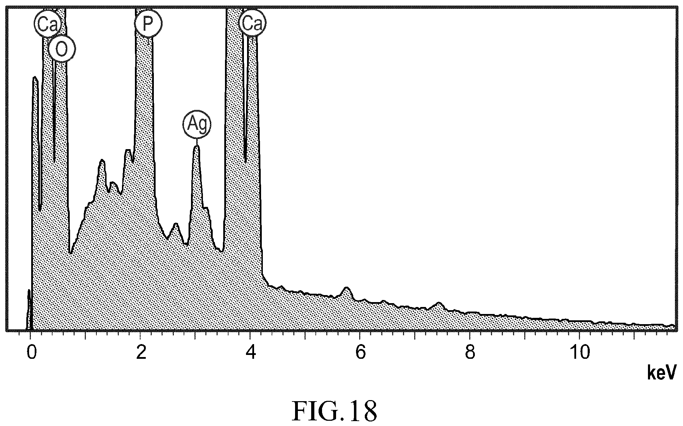

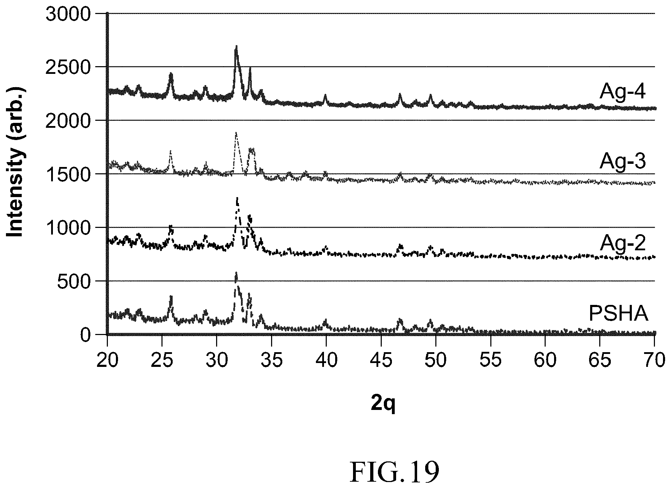

Three containers were prepared following the above protocol for three trials (see Table 13). The first container was placed in an oven at 60 °C 24 hours (denoted Ag-2 below). The second container was placed in an oven at 120 °C for 4 hours (denoted Ag-3 below). The third container was placed at room temperature for 24 hours (denoted Ag-4 below). Next, the containers were removed from the ovens and cooled to room temperature. The Ag treated PSHA coupons were washed with DI water. The Ag treated coupons were then dried at 60 °C for 2 hours before being analyzed with EDX and XRD. Fig. 16 is an EDX pattern of the Ag-2 coating. Fig. 17 is an EDX pattern of the Ag-3 coating. Fig. 18 is an EDX pattern of the Ag-4 coating. Fig. 19 is an XRD pattern of an untreated PSHA coating (denoted PSHA) and the Ag treated PSHA coatings (denoted Ag-2, Ag-3, and Ag-4). The XRD shows that no new phases were detected after ion incorporation for Ag-4 and Ag-2. Table 13 provides the experimental conditions for each of the three trials. Table 14 shows the chemical composition of the PSHA coupons treated with the Silver solution at different conditions. Table 15 shows the results of an EDX analysis and the atomic percent of the chemical components in the three PSHA treated coupons with Silver solution in various conditions as shown in Table 13.

**Table 13: Treatment conditions**

| **Coupon** | **Initial pH** | **Conditions** | **Initial Weight** | **Final Weight** |
|---|---|---|---|---|
| Ag-2 | 5.707 | 60 °C for 24 h | 7.0481 g | 7.0492 g |
| Ag-3 | 5.500 | 120 °C for 4 h | 7.0601 g | 7.0626 g |
| Ag-4 | 5.845 | 25 °C for 24 h | 7.0610 g | 7.0614 g |

**Table 14: Weight percent of the treated coating**

| **Coupon** | **P (wt%)** | **Ca (wt%)** | **O (wt%)** | **Ag (wt%)** |
|---|---|---|---|---|
| Ag-2 | 16.6 | 36.2 | 39.4 | 7.8 |
| Ag-3 | 15.2 | 30.5 | 34.5 | 19.8 |
| Ag-4 | 18.9 | 40.4 | 38.1 | 2.7 |

**Table 15: Atomic percentage of the treated coating**

| **Solution** | **P (atomic %)** | **Ca (atomic %)** | **O (atomic %)** | **Ag (atomic %)** |
|---|---|---|---|---|
| Ag-2 | 13.50 | 22.71 | 61.96 | 1.83 |
| Ag-3 | 13.64 | 21.23 | 60.00 | 5.13 |
| Ag-4 | 15.16 | 25.05 | 59.17 | 0.63 |

### Example 7: Incorporating Zn and Cu into a PSHA Coating.

To prepare a PiZn solution, 9 mL of Pi concentrate was mixed with 0.275 mL 6N NaOH. Then 1 mL 100 mM Zn(N03)2 was added. The final zinc concentration of the PiZn solution was 10 mM. To prepare 0.081 mM copper chloride solution, approximately, 0.0138 grams of cupric chloride were mixed with 100 mL of DI water. To prepare 0.1 mM calcium nitrate solution, approximately, 0.0236 grams of calcium nitrate were mixed with 100 mL of DI water. To prepare a Copper solution about 4 mL of 0.081 mM copper chloride solution and 4 mL of 0.1 mM calcium nitrate solution were mixed with 32 mL of DI water.

Two trials were prepared noted as Dual-Ion 1 and Dual-Ion 2 (see Table 16). For the first treatment, a one inch by one and a quarter inch PSHA-coated coupon was placed at the bottom of a titanium alloy container with the PSHA coating facing up. Then, 10 mL of the PiZn solution was added to submerge the PSHA coated coupon. The solution volume was roughly 70% of the container's inner volume. The container was then sealed with a titanium alloy screw cap. An O-ring were used to create a tight seal.

The sealed container was placed in an oven at 150 °C for 24 hours. The container was removed from the oven and cooled to room temperature. The PiZn treated PSHA coating was washed with DI water. The PiZn treated PSHA coating was then dried at 60 °C for 2 hours before the second treatment.

Then, for the second treatment, the PiZn treated PSHA coating (Dual-Ion 1) was subjected to a copper treatment. The PiZn treated PSHA coating was placed in the Cu solution at the bottom of a NALGENE jar with the PiZn treated PSHA coating facing up. The final Cu solution volume was about 40 mL. The container was sealed with an appropriate NALGENE jar lid.

The sealed NALGENE jar was placed in an oven at 120 °C for 4 hours. The container of the Cu solution was removed from the oven and allowed to cool to room temperature. The Zn-Cu treated PSHA coating was washed with DI water. Zn-Cu treated PSHA coating was then dried at 60 °C for 2 hours before being analyzed with EDX and XRD.

The second trial, "Dual-Ion 2," was repeated but with the reversed the order of the reactions (i.e., the Cu solution treatment was performed first followed by the PiZn solution treatment). Table 16 shows the experimental design for Dual-Ion 1 and Dual-Ion 2, and Table 17 shows the chemical composition for each sample solution of Zn and Cu incorporated into a PSHA coated coupon. Table 18 shows the atomic% of the chemical components in the Zn and Cu treated PSHA coatings (i.e., Dual-Ion 1 and Dual-Ion 2) provided from an EDX analysis.

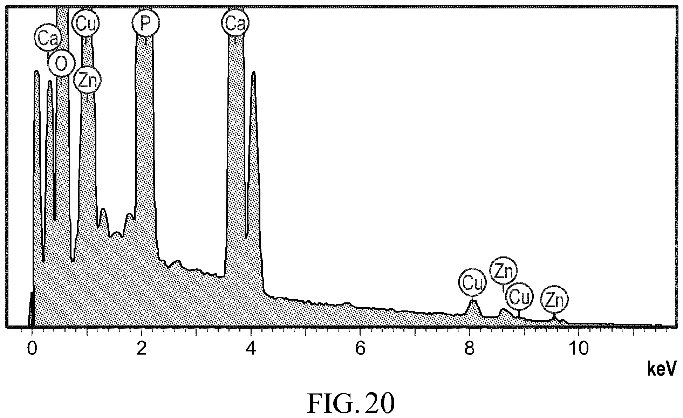

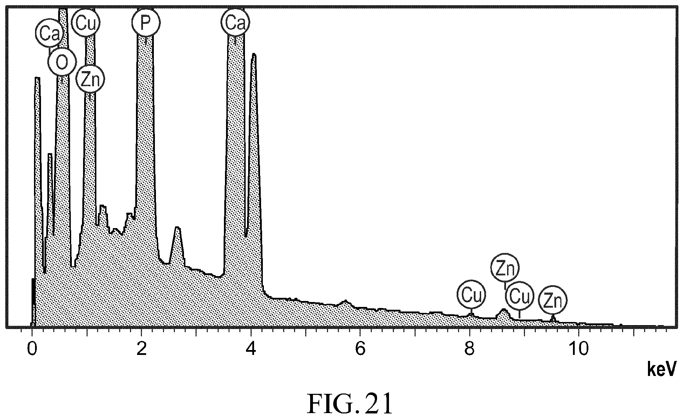

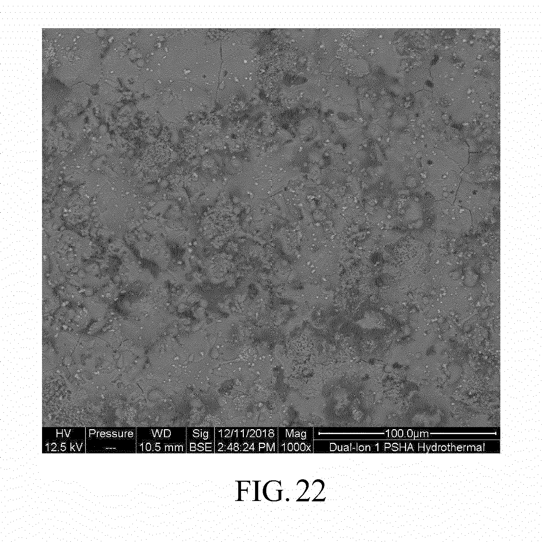

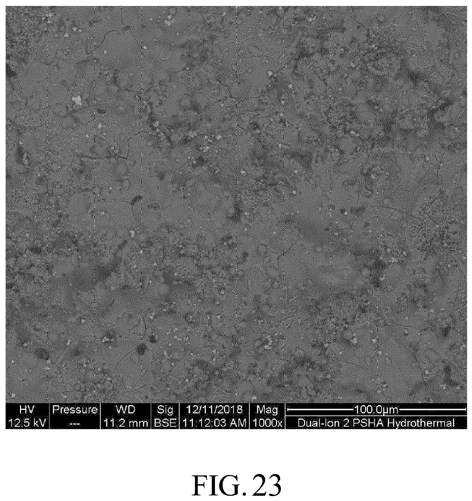

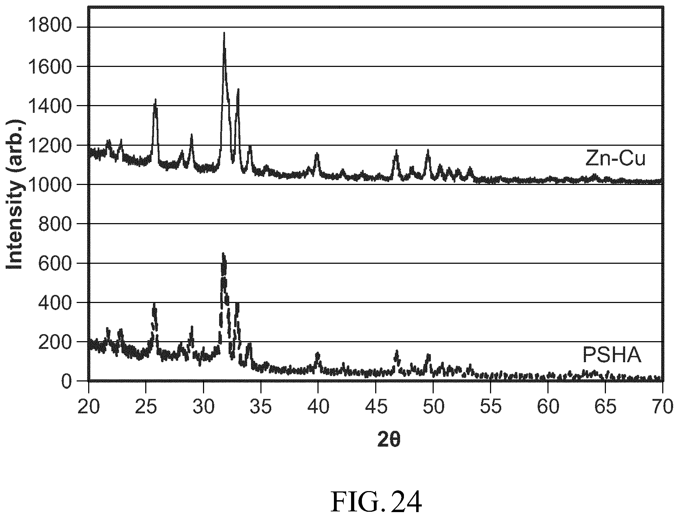

Fig. 20 is an EDX pattern of the Dual-ion 1 PSHA coating. Fig. 21 is the EDX pattern of the Dual-Ion 2 PSHA coating. The EDX of the Dual-Ion 2 did not detect copper (see Table 16). Fig. 22 and Fig. 23 are SEM images of the Dual-Ion 1 PSHA coating and the Dual-Ion 2 PSHA coating, respectively at 1000x magnification. Fig. 24 is an XRD pattern of an untreated PSHA coating (denoted PSHA) and the Zn-Cu PSHA coating (Dual-Ion 1) (denoted Zn-Cu).

**Table 16: Treatment conditions**

| **Coupon** | **First Treatment (FT)** | **Second Treatment** | **Initial Weight** | **After FT Weight** | **Final Weight** |
|---|---|---|---|---|---|
| Dual Ion-1 | PiZn solution 150 °C for 24h | Copper solution 120 °C for 4h | 7.0574 g | 7.0588 g | 7.0586 g |
| Dual Ion-2 | Copper solution 120 °C for 4h | PiZn solution 150 °C for 24h | 7.0442 g | 7.0443 g | 7.0453 g |

**Table 17: Weight percent of the treated coating**

| **Coupon** | **P (wt%)** | **Ca (wt%)** | **O (wt%)** | **Cu (wt%)** | **Zn (wt%)** |
|---|---|---|---|---|---|
| Dual-Ion 1 | 14.8 | 32.0 | 44.8 | 4.7 | 3.7 |
| Dual-Ion 2 | 15.8 | 34.1 | 46.2 | - | 5.2 |

**Table 18: Atomic percentage of the treated coating**

| **Solution** | **P (atomic %)** | **Ca (atomic %)** | **O (atomic %)** | **Zn (atomic %)** | **Cu (atomic %)** |
|---|---|---|---|---|---|
| Dual-Ion 1 | 11.35 | 18.98 | 66.58 | 1.35 | 3.72 |
| Dual-Ion 2 | 11.85 | 19.75 | 67.02 | 1.84 | - |

### Example 8: Antibacterial properties of PSHA-Zn

An overnight culture of *S. Aureus* was grown in 100% TSB medium at 36.5 °C in an orbital shaker. Then the bacteria were diluted 100X with PBS to achieve 10⁷ CFU/ml.

Bactericidal experiment tested using 1% TSB (tryptic soy broth). A PSHA-Zn coated coupon with 9.82 wt% zinc in the doped portion or a PSHA coated coupon was put into a 50 mL Falcon tube. 10 mL 1% TBS was then added to the Falcon tube. 0.1 mL of 10⁷ CFU/ml S. *Aureus* was added to the Falcon tube with so that inoculation was 10⁵ CFU/mL. The tubes with the PSHA coated coupon (Control) or the PSHA-Zn coated coupon (PSHA-Zn) were incubated for 24 h at 36.5 °C.

After 24 h at 36.5 °C, a 0.1 mL suspension was collected diluted appropriately by serial dilution in PBS. The bacteria were plated and counted using 3M Petrifilm Aerobic Count Plates: 0.75 mL of bacterial suspension was pipetted in the center of the Petrifilm and the Petrifilm was incubated for 24 h at 36.5 °C. The average of three samples for each of the control and the PSHA-Zn coating are shown in Table 19.

**Table 19: Bacterial counts**

| **Time** | **Control (CFU/mL)** | **PSHA-Zn (CFU/mL)** |
|---|---|---|
| 24 hours | 5.5 x 10⁶ | 3.7 x 10³ |

### Example 9: Crystallinity Quantification

The Panalytical X'pert Pro X-ray diffractometer employing CuKα filtered radiation and a graphite secondary monochromator was used for X-ray diffraction (XRD) studies of the particles in the suspension (centrifuged and dried) and in the films (dried and crushed). The XRD scan rate was fixed at 1°/min and the step size was 0.02°. The accelerating voltage and current used were 45 KV and 40 mA, respectively.

Quantitative analysis of the XRD data was carried out by Rietveld full profile fitting using MAUD crystallographic computation program (Ver. 2.04). Details of which are reported elsewhere (Kumar et al. 2004; Kumar et al. 2005; McCusker et al. 1999). The HA crystal model was built using the information from the International Crystal Structure Database (ICSD) for HA. The details of the HA crystallographic model is listed in Table 18. Peak shapes were modeled using the pseudo-Voigt function and two asymmetry parameters were refined. In each case four background parameters, a scale factor, five peak shape parameters, 2θ offset (zero point correction), sample displacement, cell parameters and atomic positions were refined. After the refinement of parameter, the atomic positions occupancy and thermal vibration factor for the various atomic species were refined till convergence was reached. The occupancy of the oxygen and hydrogen atoms associated with the -OH group was refined as a group (i.e., OH⁻ occupancy) using the same reasoning given by Knowles *et al.* (1994).

**Table 20. Summary of crystal parameters used for Rietveld refinement**

| Phase | Crystal system | Space Group | Lattice parameters, nm | |
|---|---|---|---|---|
| | | | a | c |
| HA | Hexagonal | P6₃/m | 0.9422 | 0.6885 |
| Atomic Coordinates | | | | |

| Atom/Ion | | x | y | z |
|---|---|---|---|---|
| Ca1 | | 0.333 | 0.667 | 0.001 |
| Ca2 | | 0.246 | 0.993 | 0.250 |
| P | | 0.400 | 0.369 | 0.250 |
| O1 | | 0.329 | 0.484 | 0.250 |
| O2 | | 0.589 | 0.466 | 0.250 |
| O3 | | 0.348 | 0.259 | 0.073 |
| OH⁻ | | 0.000 | 0.000 | 0.180 |

A PSHA coupon was prepared according to Example 1. The PSHA coated coupon was analyzed using XRD, as described above. The resulting crystallinity percentages are shown in Table 21. A PSHA coupon was prepared according to Example 7. The treated PSHA coupon was analyzed using XRD, as described above. The resulting crystallinity percentages are shown in Table 22.

**Table 21: Crystallinity Percentage Prior to Treatment**

| **Phase** | **Hydroxyapatite (%)** | **a-tricalcium phosphate (TCP) (%)** | **CaO (%)** | **b-TCP (%)** | **Amorphous calcium phosphate (ACP) (%)** |
|---|---|---|---|---|---|
| Content | 83.4 | 3.4 | UD* | 1.3 | 11.8 |

**Table 22: Crystallinity Percentage Post Treatment**

| **Phase** | **Hydroxyapatite (%)** | **a-tricalcium phosphate (TCP) (%)** | **CaO (%)** | **b-TCP (%)** | **Amorphous calcium phosphate (ACP) (%)** |
|---|---|---|---|---|---|
| Content | 97.4 | 0.7 | UD | 0.1 | 1.6 |

A non-limiting clause list is provided.
Clause 1. A coated implant comprising,
   a substrate having a bone-facing surface, and
   a doped coating located on the bone-facing surface of the substrate,
   wherein the doped coating comprises calcium and a dopant metal, and
   wherein the concentration of the dopant metal is anisotropic in the doped coating.
Clause 2. The coated implant of clause 1, wherein the dopant metal is selected from the group consisting of magnesium, strontium, gallium, zinc, copper, silver, europium, terbium, and combinations thereof.
Clause 3. The coated implant of clause 2 or 3, comprising at least two dopant metals.
Clause 4. The coated implant of any of the preceding clauses, wherein the dopant metal comprises zinc.
Clause 5. The coated implant of any of the preceding clauses, wherein the doped coating comprises hydroxyapatite.
Clause 6. The coated implant of any of the preceding clauses, wherein the doped coating comprises a doped portion and an undoped portion located between the doped portion and the substrate.
Clause 7. The coated implant of clause 6, wherein the dopant metal is at least 0.5% by weight of the doped portion of the doped coating.
Clause 8. The coated implant of clause 6 or 7, wherein the dopant metal is at least 0.1 atomic percent of the doped portion of the doped coating.
Clause 9. The coated implant of any of clauses 6-8, wherein the dopant metal is about 0.1 atomic percent to about 6 atomic percent of the doped portion of the doped coating.
Clause 10. The coated implant of any of clauses 6-9, wherein the doped portion of the doped coating comprises less than about 40% calcium by weight.
Clause 11. The coated implant of any of clauses 6-10, wherein the doped portion of the doped coating comprises less than about 35% calcium by weight.
Clause 12. The coated implant of any of clauses 6-11, wherein the dopant metal is at least 0.5% by weight of the doped portion of the doped coating.
Clause 13. The coated implant of any of clauses 6-12, wherein the dopant metal is at least 2% by weight of the doped portion of the doped coating.
Clause 14. The coated implant of any of clauses 6-13, wherein the concentration of calcium is higher in the undoped portion than in the doped portion.
Clause 15. The coated implant of any of the preceding clauses, wherein the doped coating is at least about 95% crystalline.
Clause 16. A process for forming a coated implant, the process comprising
   contacting a hydroxyapatite coating on a substrate with an aqueous solution comprising a dopant metal ion, and
   washing the hydroxyapatite coating after the step of contacting to form a doped hydroxyapatite coating comprising calcium and a dopant metal,
   wherein the concentration of the dopant metal is anisotropic in the doped hydroxyapatite coating.
Clause 17. The process of clause 16, wherein the contacting step is performed at a temperature of about 25°C to about 175°C.
Clause 18. The process of clause 16 or 17, wherein the contacting step is performed for a time of about 30 minutes to about 80 hours.
Clause 19. The process of any of clauses 16-18, wherein the contacting step is performed for a time of about 1.5 hours to about 72 hours.
Clause 20. The process of any of clauses 16-19, wherein the contacting step is performed for about 24 hours at about 25°C.
Clause 21. The process of any of clauses 16-19, wherein the aqueous solution has a pH ranging from about 5.5 to about 8.0.
Clause 22. The process of any of clauses 16-20, wherein the contacting step is performed at a temperature of about 25°C to about 60°C.
Clause 23. The process of any of clauses 16-20, wherein the contacting step is performed at a temperature of about 60°C to about 150°C .
Clause 24. The process of any of clauses 16-20, wherein the contacting step is performed at a temperature of about 60°C to about 120°C .
Clause 25. The process of any of clauses 16-24, wherein the dopant metal ion is selected from the group consisting of magnesium, strontium, gallium, zinc, copper, silver, europium, terbium, and combinations thereof.
Clause 26. The process of any of clauses 16-25, wherein the aqueous solution comprises at least two dopant metal ions.
Clause 27. The process of any of clauses 16-26, wherein the percentage by weight of calcium in the doped hydroxyapatite coating is less than the percentage by weight of calcium prior to the step of contacting.
Clause 28. The process of any of clauses 16-27, wherein the doped hydroxyapatite coating has improved antibacterial properties compared to the hydroxyapatite coating.
Clause 29. The process of clause 28, wherein the improvement is at least 10-fold.
Clause 30. The process of clause 29, wherein the improvement is at least 100-fold.
Clause 31. The process of clause 30, wherein the improvement is at least 1,000-fold.
Clause 32. The process of any of clauses 16-31, wherein the percent crystallinity of the doped hydroxyapatite coating is higher than the percent crystallinity of the hydroxyapatite coating.
Clause 33. The process of clause 32, wherein the percent crystallinity after the step of contacting is at least about 95% crystalline.
Clause 34. A process for forming a doped coating on an implant, the process comprising
   exchanging lattice positions from a hydroxyapatite coating with a dopant metal to form a doped hydroxyapatite coating comprising a doped hydroxyapatite portion and an undoped hydroxyapatite portion, and
   washing the doped hydroxyapatite coating,
   wherein the concentration of the dopant metal is anisotropic in the doped hydroxyapatite coating.
Clause 35. The process of clause 34, wherein the step of exchanging is performed by contacting the hydroxyapatite-coated substrate with an aqueous solution comprising a dopant metal ion.
Clause 36. The process of clause 34 or 35, wherein the percent crystallinity of the doped hydroxyapatite coating is greater than the percent crystallinity of the hydroxyapatite coating.
Clause 37. The process of any of clauses 34-36, wherein the percent crystallinity after the step of contacting is at least about 95% crystalline.
Clause 38. A coated implant comprising,
   a substrate, and
   a coating located on a surface of the substrate,
   wherein the coating includes a doped hydroxyapatite portion and an undoped hydroxyapatite portion located between the doped hydroxyapatite portion and the substrate, and
   wherein the doped hydroxyapatite portion comprises calcium and a dopant metal.
Clause 39. The coated implant of clause 38, wherein the dopant metal is selected from the group consisting of magnesium, strontium, gallium, zinc, copper, silver, europium, terbium, and combinations thereof.
Clause 40. The coated implant of clause 38 or 39, wherein the undoped hydroxyapatite portion does not include the dopant metal.
Clause 41. The coated implant of any of clauses 38-40, wherein the coating comprises calcium and zinc.
Clause 42. The coated implant of any of clauses 38-40, wherein zinc is not present in the undoped hydroxyapatite portion.
Clause 43. A coated implant comprising,
   a substrate having a bone-facing surface, and
   a coating located on the bone-facing surface of the substrate, the coating having an outer surface opposite the substrate,
   wherein the coating comprises calcium and a dopant metal, and the concentration of the dopant metal decreases from the outer surface of the coating toward the surface of the substrate.
Clause 44. The coated implant of clause 43, wherein the dopant metal is selected from the group consisting of magnesium, strontium, gallium, zinc, copper, silver, europium, terbium, and combinations thereof.
Clause 45. The coated implant of clause 43 or 44, wherein the dopant metal is zinc.

## Claims

1. A coated implant comprising,
a substrate having a bone-facing surface, and
a doped coating located on the bone-facing surface of the substrate,
wherein the doped coating comprises calcium and a dopant metal, and
wherein the concentration of the dopant metal is anisotropic in the doped coating.

2. The coated implant of claim 1, wherein the dopant metal is selected from the group consisting of magnesium, strontium, gallium, zinc, copper, silver, europium, terbium, and combinations thereof.

3. The coated implant of claim 2, comprising at least two dopant metals.

4. The coated implant of claim 2, wherein the dopant metal comprises zinc.

5. The coated implant of claim 4, wherein the doped coating comprises hydroxyapatite.

6. The coated implant of claim 1, wherein the doped coating comprises a doped portion and an undoped portion located between the doped portion and the substrate.

7. The coated implant of claim 6, wherein the dopant metal is at least 0.5% by weight of the doped portion of the doped coating.

8. The coated implant of claim 1, wherein the doped coating is at least about 95% crystalline.

9. A process for forming a coated implant, the process comprising contacting a hydroxyapatite coating on a substrate with an aqueous solution comprising a dopant metal ion, and
washing the hydroxyapatite coating after the step of contacting to form a doped hydroxyapatite coating comprising calcium and a dopant metal,
wherein the concentration of the dopant metal is anisotropic in the doped hydroxyapatite coating.

10. The process of claim 9, wherein the contacting step is performed at a temperature of about 25°C to about 175°C.

11. The process of claim 9, wherein the aqueous solution has a pH ranging from about 5.5 to about 8.0.

12. The process of claim 9, wherein the contacting step is performed for a time of about 30 minutes to about 80 hours.

13. The process of claim 9, wherein the doped hydroxyapatite coating comprises a doped portion and an undoped portion located between the doped portion and the substrate, and the dopant metal is not present in the undoped portion.

14. The process of claim 9, wherein the dopant metal ion is selected from the group consisting of magnesium, strontium, gallium, zinc, copper, silver, europium, terbium, and combinations thereof.

15. The process of claim 9, wherein the doped hydroxyapatite coating has improved antibacterial properties compared to the hydroxyapatite coating.

16. The process of claim 15, wherein the improvement is at least 10-fold.

17. The process of claim 9, wherein the percent crystallinity of the doped hydroxyapatite coating is higher than the percent crystallinity of the hydroxyapatite coating.

18. A coated implant comprising,
a substrate, and
a coating located on a surface of the substrate,
wherein the coating includes a doped hydroxyapatite portion and an undoped hydroxyapatite portion located between the doped hydroxyapatite portion and the substrate, and
wherein the doped hydroxyapatite portion comprises calcium and a dopant metal.

19. The coated implant of claim 18, wherein the dopant metal is selected from the group consisting of magnesium, strontium, gallium, zinc, copper, silver, europium, terbium, and combinations thereof.

20. The coated implant of claim 18, wherein the undoped hydroxyapatite portion does not include the dopant metal.
